# EUROPEAN PATENT APPLICATION

(11) **EP 3 878 936 A1**
(43) Date of publication of application: **15.09.2021**
(21) Application number: 19881623.3
(22) Date of filing: 07.11.2019
(51) Int. Cl.: C12M 1/00, C12Q 1/6844, C12Q 1/6881, G01N 33/15, G01N 33/50

(54) **METHOD FOR DETECTING GENOME-RELATED INFORMATION OF CELL COEXISTING WITH ONE OR MORE SUBSTANCES TO BE DETECTED**

(30) Priority: 07.11.2018 JP 2018210012
(71) Applicant: The University of Tokyo, Bunkyo-ku, Tokyo 113-8654 (JP); RIKEN, Wako-shi Saitama 351-0198 (JP)
(72) Inventor: YACHIE Nozomu, Tokyo 113-8654 (JP); OTA Sadao, Tokyo 113-8654 (JP); KAWASAKI Fumiko, Wako-shi, Saitama 351-0198 (JP)
(74) Representative: De Clercq & Partners
(86) International application number: PCT/JP2019/043762
(87) International publication number: WO 2020/096015

(57) **Abstract**

The present invention provides a method for detecting genome-related information of a cell coexisting with at least one type of test substance, for various types of test substances. More specifically, the present invention provides a method for detecting genome-related information of a cell or a derivative thereof coexisting with at least one type of test substance, including:
preparing a plurality of types of first compartments that contain at least one type of test substance and a first barcode nucleic acid, and a plurality of types of second compartments that contain a cell or a derivative thereof and a second barcode nucleic acid-linked bead;
forming a plurality of types of third compartments in which one first compartment and one second compartment are fused;
hybridizing each of the genome-related nucleic acid and the first barcode nucleic acid with a second barcode nucleic acid to obtain a hybridized complex;
producing an amplified product derived from the hybridized complex; and
detecting genome-related information in the cell after coexistence with the test substance using an expression pattern of the amplified product as an index.

SEQUENCE LISTING

<110>  Tokyo University
       Institute of Physical and Chemical Research

<120>  Method of Detecting Genome-related Information of a cell existed
       together with one or more test substances

<130>  230284PX

<150>  JP2018-210012
<151>  2018-11-07

<160>  8

<170>  PatentIn version 3.5

<210>  1
<211>  41
<212>  DNA
<213>  Artificial

<220>
<223>  Nucleic acids in Linker

<400>  1
gtaacgatgg agctgtcact tggaattctc gggtgccaag g                          41


<210>  2
<211>  20
<212>  DNA
<213>  Artificial

<220>
<223>  Nucleic acids in Linker

<400>  2
agtgacagct ggatcgttac                                                  20


<210>  3
<211>  59
<212>  DNA
<213>  Artificial

<220>
<223>  Primer

<400>  3
ccttggcacc cgagaattcc accacaatga aaaaaaaaaa aaaaaaaaaa aaaaaaaaa          59


<210>  4
<211>  59
<212>  DNA
<213>  Artificial

<220>
<223>  Primer

<400>  4
ccttggcacc cgagaattcc atgagaccta aaaaaaaaaa aaaaaaaaaa aaaaaaaaa          59

<210>  5
<211>  19
<212>  DNA
<213>  Artificial

<220>
<223>  Primer

<400>  5
cttggcaccc gagaattcc                                                    19


<210>  6
<211>  58
<212>  DNA
<213>  Artificial

<220>
<223>  Primer

<400>  6
aatgatacgg cgaccaccga gatctacact ctttccctac acgacgctct tccgatct         58


<210>  7
<211>  63
<212>  DNA
<213>  Artificial

<220>
<223>  Primer

<400>  7
caagcagaag acggcatacg agatattggc gtgactggag ttccttggca cccgagaatt       60

cca                                                                     63


<210>  8
<211>  63
<212>  DNA
<213>  Artificial

<220>
<223>  Primer

<400>  8
caagcagaag acggcatacg agattacaag gtgactggag ttccttggca cccgagaatt       60

cca                                                                     63

## Description

### CROSS-REFERENCE TO RELATED APPLICATIONS

This application is based upon and claims the benefit of priority from Japanese Patent Application No. 2018-210012, filed on November 7, 2018; the entire contents of which are incorporated herein by reference.

### BACKGROUND OF THE INVENTION

### Field of the Invention

The present invention relates to a method for effectively detecting genome-related information of a cell coexisting with at least one type of test substance, for various types of test substances.

### Background Art

Phenotype screening is known as multidrug screening using cells. Phenotype screening is a technique of searching for a drug (for example, a low molecular compound, a peptide, etc.) that changes the phenotype of cells or organs, for example, cell proliferation rate or cell death, as an index. However, the information that can be obtained by phenotype screening is limited to the cell proliferation rate and cell death, and no further information, especially genetic information of cells, can be obtained. In addition, it has been difficult to increase the number of types of drugs per screening and to effectively identify phenotype information in the conventional phenotype screening.

In addition, conventionally, phenotype detection has been performed only in cell populations. However, recent studies have revealed that gene expression varies and is diverse from cell to cell even in similar cell types such as cancer tissues, and there is a need for detecting gene expression, etc., of each cell.

For this purpose, at present, as a method for detecting transcription products derived from one cell, a method for obtaining data on genetic information of cells using beads to which oligonucleotides containing barcode sequences are bound and using sequencing techniques is known. (Patent Literature 1, Non Patent Literature 1)

However, there has been no report on obtaining information of single cells in multidrug screening.

Under such technical circumstances, it can be said that there is a need for a means of effectively detecting genome-related information of cells obtained from cells coexisting with at least one type of test substance, for various types of test substances.

### Citation List

### Non Patent Literature

Non Patent Literature 1
E. Z. Macosko et al., Highly Parallel Genome-wide Expression Profiling of Individual Cells Using Nanoliter Droplets. Cell. 161, 1202-1214 (2015)

### Patent Literature

Patent Literature 1
WO 2015/166768 A

### SUMMARY OF THE INVENTION

The present invention provides a method for effectively detecting genome-related information of a cell or derivative thereof coexisting with at least one type of test substance, for various types of test substances.

The present inventors have now found that, when each one of a plurality of types of compartments containing at least one type of a test substance and a barcode nucleic acid corresponding to the test substance is fused with each one of a plurality of compartments containing a cell or a derivative thereof and a barcode nucleic acid-linked bead to form a compartment, so that the cell or derivative thereof coexists with the test substance, and then genome-related information is measured and associated, genome-related information of the cell or derivative thereof coexisting with the at least one type of test substance can be effectively detected, for various types of test substances. Further, the present inventors have found that when a barcode nucleic acid-containing bead having imaging information that can be distinguished from each other is further incorporated, and nondestructive measurement information of the cell is associated with imaging information of the bead, and then genome-related information is measured and associated, nondestructive measurement information and genome-related information of the cell can be integrally and effectively detected. The present invention is based on the findings.

The present invention encompasses the following inventions.
[1] A method for detecting genome-related information of a cell or a derivative thereof coexisting with at least one type of test substance, including:
   preparing a plurality of target compartments containing a cell or a derivative thereof coexisting with the test substance, a first barcode nucleic acid, and a second barcode nucleic acid-linked bead,
   wherein the first barcode nucleic acid is preliminarily associated with the test substance, and
   the second barcode nucleic acid-linked bead contains a plurality of second barcode nucleic acids hybridizable with a genome-related nucleic acid corresponding to a cell genome or a derivative thereof or the first barcode nucleic acid;
   hybridizing each of the genome-related nucleic acid and the first barcode nucleic acid with the second barcode nucleic acid to obtain a hybridized complex;
   producing an amplified product derived from the hybridized complex; and
   detecting genome-related information in the cell after coexistence with the test substance using an expression pattern of the amplified product as an index.
[2] The method according to [1], wherein an association of the first barcode nucleic acid with the test substance includes making the test substance coexist with the cell or derivative thereof and the first barcode nucleic acid.
[3] The method according to [1] or [2], wherein a preparation of the target compartments includes binding a subcompartment that contains the cell or derivative thereof made to coexist with the test substance and the first barcode nucleic acid, to a subcompartment that contains the second barcode nucleic acid-linked bead.
[4] A method for detecting genome-related information of a cell or a derivative thereof coexisting with at least one type of test substance, including:
   preparing a plurality of types of first compartments that contain at least one type of test substance and a first barcode nucleic acid corresponding to the test substance, and a plurality of types of second compartments that contain a cell or a derivative thereof and a second barcode nucleic acid-linked bead, respectively,
   wherein the second barcode nucleic acid-linked bead is linked to a plurality of second barcode nucleic acids hybridizable with a genome-related nucleic acid corresponding to a cell genome or a derivative thereof or the first barcode nucleic acid;
   forming a plurality of types of third compartments in which one first compartment and one second compartment are fused,
   wherein the cell or derivative thereof coexists with the test substance in the third compartments;
   hybridizing each of the genome-related nucleic acid and the first barcode nucleic acid with the second barcode nucleic acid to obtain a hybridized complex;
   producing an amplified product derived from the hybridized complex; and
   detecting genome-related information in the cell after coexistence with the test substance using an expression pattern of the amplified product as an index.
[5] The method according to any one of claims 1 to 4, wherein the test substance is identified using an expression pattern of a first amplified product derived from a hybridized complex of the first barcode nucleic acid with the second barcode nucleic acid as an index, and
   the genome-related information of the cell is detected using an expression pattern of a second amplified product derived from a hybridized complex of the genome-related nucleic acid with the second barcode nucleic acid as an index.
[6] The method according to any one of [1] to [5], wherein the genome-related nucleic acid is the genome DNA of the cell, or an RNA derived from the genome DNA of the cell or a cDNA thereof.
[7] The method according to any one of [1] to [6], wherein each first barcode nucleic acid includes a first common barcode region which is common in same test substance and a first hybridize region hybridizable with the second barcode nucleic acid.
[8] The method according to any one of [1] to [7], wherein sequence information of the first common barcode region becomes an index for identifying the test substance.
[9] The method according to any one of [1] to [8], wherein each of the plurality of second barcode nucleic acids linked to the second bead includes a second common barcode region which is in common with each other, a second unique barcode region which can be clearly distinguished from each other, and a second hybridize region hybridizable with the genome-related nucleic acid or the first barcode nucleic acid.
[10] The method according to [8], wherein sequence information of the second common barcode region becomes an index for identifying the cell or a derivative thereof existing in the compartment.
[11] The method according to [9] or [10], wherein sequence information of the second unique barcode region becomes an index for identifying the genome-related nucleic acid.
[12] The method according to any one of [1] to [11], wherein the second barcode nucleic acid further includes a PCR primer region.
[13] The method according to any one of [9] to [12], wherein the second hybridize region includes the first hybridize region or a nucleic acid complementary to the genome-related nucleic acid.
[14] The method according to any one of [1] to [3] for integrally detecting nondestructive measurement information and genome-related information of a cell coexisting with at least one type of test substance,
   the target compartment further containing a bead having a third barcode nucleic acid,
   wherein each bead having a third barcode nucleic acid is:
      a particle cleavably linked to a third barcode nucleic acid corresponding to each imaging information or
      an organism containing a third barcode nucleic acid corresponding to each imaging information, and
   imaging information of the bead having a third barcode nucleic acid can be clearly distinguished from each other,
   the method further including:
      detecting both nondestructive measurement information of the cell and imaging information of the bead having a third barcode nucleic acid and associating the nondestructive measurement information of the cell with the imaging information of the bead having a third barcode nucleic acid;
      cleaving or taking out the third barcode nucleic acid from the associated bead having the third barcode nucleic acid, and hybridizing each of the genome-related nucleic acid and the third barcode nucleic acid with the second barcode nucleic acid to obtain a hybridized complex;
      producing an amplified product derived from the hybridized complex; and
      integrally detecting nondestructive measurement information and genome-related information of the cell using an expression pattern of the amplified product as an index.
[15] The method according to any one of [4] to [13] for integrally detecting nondestructive measurement information and genome-related information of a cell coexisting with at least one type of test substance,
   the second compartment further containing a bead having a third barcode nucleic acid,
   wherein the bead having a third barcode nucleic acid is:
      a particle cleavably linked to a third barcode nucleic acid corresponding to each imaging information or
      an organism containing a third barcode nucleic acid corresponding to each imaging information, and
   imaging information of the bead having a third barcode nucleic acid in the third compartment can be clearly distinguished from each other, and
   the method further including:
      detecting both nondestructive measurement information of the cell and imaging information of the bead having a third barcode nucleic acid and associating the nondestructive measurement information of the cell with the imaging information of the bead having a third barcode nucleic acid;
      cleaving or taking out the third barcode nucleic acid from the associated bead having the third barcode nucleic acid, and hybridizing each of the genome-related nucleic acid and the third barcode nucleic acid with the second barcode nucleic acid to obtain a hybridized complex;
      producing an amplified product derived from the hybridized complex; and
      integrally detecting nondestructive measurement information and genome-related information of the cell using an expression pattern of the amplified product as an index.
[16] The method according to [14] or [15], wherein both nondestructive measurement information of the cell and imaging information of the bead having a third barcode nucleic acid are detected, and the nondestructive measurement information of the cell is associated with the imaging information of the bead having the third barcode nucleic acid, before the preparation of the third compartment, in the second compartment and/or the third compartment.
[17] The method according to any one of [14] to [16], wherein the nondestructive measurement information is based on at least one piece of measurement information selected from color, fluorescence, size, shape, electromagnetic wave, transmission, phase, scattering, reflection, coherent Raman, Raman, and absorption spectrum.
[18] A combination of a first barcode nucleic acid and a second barcode nucleic acid-linked bead for detecting genome-related information of a cell coexisting with at least one type of test substance, wherein
   each first barcode nucleic acid corresponds to the at least one type of test substance coexisting with the cell;
   the second barcode nucleic acid-linked bead is linked to a plurality of second barcode nucleic acids hybridizable with a genome-related nucleic acid corresponding to a cell genome or a derivative thereof or the first barcode nucleic acid; and
   the test substance can be identified using an expression pattern of a first amplified product derived from a hybridized complex of the first barcode nucleic acid with the second barcode nucleic acid as an index, and the genome-related information of the cell can be detected using an expression pattern of a second amplified product derived from a hybridized complex of the genome-related nucleic acid with the second barcode nucleic acid as an index.
[19] A detecting agent containing a first barcode nucleic acid, which is used together with a second barcode nucleic acid-linked bead, for detecting genome-related information of a cell coexisting with at least one type of test substance, wherein
   each first barcode nucleic acid corresponds to the at least one type of test substance coexisting with the cell;
   the second barcode nucleic acid-linked bead is linked to a plurality of second barcode nucleic acids hybridizable with a genome-related nucleic acid corresponding to a cell genome or a derivative thereof or the first barcode nucleic acid; and
   the test substance can be identified using an expression pattern of a first amplified product derived from a hybridized complex of the first barcode nucleic acid with the second barcode nucleic acid as an index, and the genome-related information of the cell can be detected using an expression pattern of a second amplified product derived from a hybridized complex of the genome-related nucleic acid with the second barcode nucleic acid as an index.
[20] A detecting agent containing a second barcode nucleic acid-linked bead, which is used together with a first barcode nucleic acid, for detecting genome-related information of a cell coexisting with at least one type of test substance, wherein
   each first barcode nucleic acid corresponds to the at least one type of test substance coexisting with the cell;
   the second barcode nucleic acid-linked bead is linked to a plurality of second barcode nucleic acids hybridizable with a genome-related nucleic acid corresponding to a cell genome or a derivative thereof or the first barcode nucleic acid; and
   the test substance can be identified using an expression pattern of a first amplified product derived from a hybridized complex of the first barcode nucleic acid with the second barcode nucleic acid as an index, and the genome-related information of the cell can be detected using an expression pattern of a second amplified product derived from a hybridized complex of the genome-related nucleic acid with the second barcode nucleic acid as an index.
[21] A composition including a plurality of types of first compartments, wherein each first compartment contains one type of test substance and a first barcode nucleic acid corresponding to the one type of test substance.
[22] A composition including a plurality of types of second compartments, which are used together with a first barcode nucleic acid, for detecting genome-related information of a cell coexisting with at least one type of test substance, wherein
   each first barcode nucleic acid corresponds to the at least one type of test substance coexisting with the cell;
   each second compartment contains a cell or a derivative thereof and a second barcode nucleic acid-linked bead;
   the second barcode nucleic acid-linked bead is linked to a plurality of second barcode nucleic acids hybridizable with a genome-related nucleic acid corresponding to a cell genome or a derivative thereof or the first barcode nucleic acid; and
   the test substance can be identified using an expression pattern of a first amplified product derived from a hybridized complex of the first barcode nucleic acid with the second barcode nucleic acid as an index, and the genome-related information of the cell can be detected using an expression pattern of a second amplified product derived from a hybridized complex of the genome-related nucleic acid with the second barcode nucleic acid as an index.
[23] A method for screening for a test substance, based on the genome-related information of the cell or derivative thereof detected by the method according to [1] to [17].
[24] A method for screening for a test substance, based on the nondestructive measurement information and the genome-related information of the cell detected by the method according to [14] to [17].

According to the present invention, genome-related information of a cell or a derivative thereof coexisting with at least one type of test substance can be effectively detected for various test substances. Further, according to the present invention, nondestructive measurement information and genome-related information of cells can be integrally and effectively detected. Further, according to the present invention, information of single cells can be detected.

### BRIEF DESCRIPTION OF THE DRAWINGS

FIG. 1 is a schematic diagram on a first barcode nucleic acid.
FIG. 2 is a schematic diagram on a second barcode nucleic acid-linked bead.
FIG. 3 is a schematic diagram on a detection method.
FIG. 4 is a schematic diagram on a bead having a third barcode nucleic acid.
FIG. 5 is a schematic diagram showing another embodiment of the detection method.
FIG. 6 is a schematic diagram showing another embodiment of the first barcode nucleic acid, the second barcode nucleic acid-linked bead and the bead having a third barcode nucleic acid.
FIG. 7 is a schematic diagram showing further another embodiment of the first barcode nucleic acid, the second barcode nucleic acid-linked bead and the bead having a third barcode nucleic acid.
FIG. 8 is a schematic diagram showing another embodiment of the bead having a third barcode nucleic acid.
FIG. 9 is a schematic diagram showing another embodiment of the first barcode nucleic acid, the second barcode nucleic acid-linked bead and the bead having a third barcode nucleic acid.
FIG. 10 is a schematic diagram showing a method for producing a compartment.
FIG. 11 is a schematic diagram showing another embodiment of the method for producing a compartment.
FIG. 12 is a photograph of a droplet (compartment) in which the second barcode nucleic acid-linked bead and a cell are enclosed. The spherical body present in the center of the photograph is the second barcode nucleic acid-linked bead, and the vesicles floating around it are the cells.
FIG. 13 is a photograph of a droplet (compartment) in which the second barcode nucleic acid-linked bead, the bead having a third barcode nucleic acid and a cell are enclosed. The spherical body present in the center of the photograph is the second barcode nucleic acid-linked bead, the substances floating around the second barcode nucleic acid-linked bead are the cells, and a light-colored image present at the peripheral edge part of the droplet is the bead having a third barcode nucleic acid.

### DETAILED DESCRIPTION OF THE INVENTION

### Definition

"Genome-related information" as used herein means nucleic acid sequence information derived from a genome-related nucleic acid corresponding to a cell genome or a derivative thereof. Here, "genome-related nucleic acid" means preferably a genome DNA of a cell, an RNA such as an mRNA derived from a genome of a cell or a cDNA thereof, or a nucleic acid probe specifically interacting with (for example, binding to) a molecule such as a protein expressed in a cell (hereinafter referred to as nucleic acid probe specific to a molecule such as a protein expressed in a cell). The nucleic acid probe preferably contains a barcode nucleic acid which is cleavably linked to a molecule specifically binding to a molecule such as a target protein (hereinafter also referred to as binding molecule) and which can be clearly distinguished from each other. When the nucleic acid is a genome DNA, the DNA may be a fragment cleaved by a restriction enzyme, etc., or a DNA tag may be introduced into the DNA fragment.

As used herein, "barcode region" is not limited as long as it is a region of a base sequence containing T (thymine) or U (uracil), A (adenine), G (guanine) and C (cytosine), and is a sequence of a common barcode region or unique barcode region which will be described later. The barcode nucleic acid is a nucleic acid containing the barcode region and enables discrimination of genome-related information of a cell, a test substance coexisting with the cell, and imaging information derived from a bead.

As the types of the barcode regions, two types of regions, a common barcode region and a unique barcode region, exist.

The common barcode region is a barcode region common in the same target to be discriminated. When the target to be discriminated is a test substance, the example includes the barcode region different for each test substance, namely, a barcode region common in one test substance. Labeling with the common barcode region enables discrimination of each test substance. When the target to be discriminated is a combination of test substances, such as a case that a combination of test substances is contained in one compartment, labeling is made with a barcode nucleic acid having a barcode region different for each combination, that is, labeling is made with a barcode region common in a combination of particular test substances or a common barcode region common for each test substance. Labeling with the common barcode region enables discrimination of a combination of test substances. When the target to be discriminated is genome-related information of a single cell, the example includes a barcode region different for each cell, namely, a barcode region common in one cell. Labeling with the common barcode region enables discrimination of genome-related information derived from the same cell. When the target to be discriminated is imaging information of a bead, the example includes a barcode region different for each bead having the same imaging information, namely, a barcode region common in a bead having the same imaging information. Labeling with the common barcode region enables discrimination of imaging information derived from a bead having the same imaging information.

Furthermore, in the nucleic acid probe specific to a molecule such as a protein expressed in a cell, the nucleic acid probe contains a molecule specifically binding to a molecule such as a protein (binding molecule). Here, in the nucleic acid probe, a barcode region different for each of the binding molecules, namely, a barcode region common in the same binding molecule is also regarded as the common barcode region.

The unique barcode region enables clear distinction of each barcode nucleic acid by labeling with a barcode region different for each barcode nucleic acid. For example, labeling with the unique barcode region can discriminate a bead linked to each barcode nucleic acid, an organism containing each barcode nucleic acid, and a genome-related nucleic acid hybridized with each barcode nucleic acid.

The length of the barcode region is not particularly limited, and is preferably a sequence of 10 to 40 bases in length. For example, when the barcode region is 12 bases in length, 4¹² types of diverse barcode sequences can be subjected to nucleic acid amplification at a time, and 4¹² types of beads can be produced.

"Hybridize" as used herein means that a hybridize region of a barcode nucleic acid forms a double-stranded complex with a genome-related nucleic acid corresponding to a cell genome or a derivative thereof or another barcode nucleic acid. Exemplary conditions for forming the double-stranded complex include hybridization with 40 to 45% formamide, 1.0 M NaCI and 0.1% SDS at 37°C and washing with 0.5x to 1× SSC at 55°C to 60°C. Another embodiment of forming the double-stranded complex is, for example, formation of the complex under a stringent condition. Here, the stringent condition means a condition under which a so-called specific complex is formed and no non-specific complex is formed, and includes the above exemplary conditions. Such a stringent condition is known to a person skilled in the art, and can be set with reference to, for example, Molecular Cloning (Third Edition, Cold Spring Harbor Laboratory Press, New York) or Current protocols in molecular biology (edited by Frederick M. Ausubel et al., 1987). Examples of the sequence with which the hybridize region of the barcode nucleic acid is hybridized include a sequence complementary to the hybridize region.

Therefore, "hybridize region" is preferably a region which binds to (is hybridized with) a genome-related nucleic acid corresponding to a cell genome or a derivative thereof or another barcode nucleic acid. The hybridize region preferably coexists with the barcode region in the barcode nucleic acid.

For example, when the genome-related nucleic acid is an mRNA, a second hybridize region in a second barcode nucleic acid is preferably polythymine composed of T. The length of polythymine may be a length at which polythymine can be annealed (can be hybridized) with a polyadenine (A) tail of the mRNA. In the above case, first and third hybridize regions are each preferably a sequence complementary to polythymine, for example, polyadenine (polyA).

When the genome-related nucleic acid is a DNA such as a genome DNA, the second hybridize region in the second barcode nucleic acid preferably includes a sequence complementary to a particular sequence of the DNA or a sequence of a DNA tag introduced into the DNA. In the above case, the first and third hybridize regions are each preferably a sequence complementary to the second hybridize region.

When the nucleic acid probe specific to a molecule such as a protein expressed in a cell contains a barcode nucleic acid, and the barcode nucleic acid contains a hybridize region, the second hybridize region in the second barcode nucleic acid preferably includes a sequence complementary to the hybridize region. In the above case, the above hybridize region in the barcode nucleic acid linked to the nucleic acid probe is preferably a sequence complementary to the second hybridize region.

### Method for detecting genome-related information of cell or derivative thereof coexisting with at least one type of test substance

According to a first embodiment of the present invention, a method for detecting genome-related information of a cell or a derivative thereof coexisting with at least one type of test substance is characterized by including:
preparing a plurality of types of first compartments that contain at least one type of test substance and a first barcode nucleic acid corresponding to the test substance, and a plurality of types of second compartments that contain a cell or a derivative thereof and a second barcode nucleic acid-linked bead, respectively,
wherein the second barcode nucleic acid-linked bead is linked to a plurality of second barcode nucleic acids hybridizable with a genome-related nucleic acid corresponding to a cell genome or a derivative thereof or the first barcode nucleic acid; and
forming a plurality of types of third compartments in which one first compartment and one second compartment are fused,
wherein the cell or derivative thereof coexists with the test substance in the third compartments;
hybridizing each of the genome-related nucleic acid and the first barcode nucleic acid with the second barcode nucleic acid to obtain a hybridized complex;
producing an amplified product derived from the hybridized complex; and
detecting genome-related information in the cell after coexistence with the test substance using an expression pattern of the amplified product as an index.

The detection method according to the first embodiment of the present invention will be described for each step based on preferred embodiments, but the present invention is not particularly limited thereto.

### Step of preparing first, second and third compartments

A step of preparing a first compartment and a second compartment of the present invention is a step of preparing a plurality of types of first compartments that contain at least one type of test substance and a first barcode nucleic acid corresponding to the test substance per compartment, and a plurality of types of second compartments that contain a cell or a derivative thereof and a second barcode nucleic acid-linked bead per compartment, respectively. Here, the second barcode nucleic acid-linked bead is linked to a plurality of second barcode nucleic acids hybridizable with a genome-related nucleic acid corresponding to a cell genome or a derivative thereof or the first barcode nucleic acid.

### Compartment

The compartment of the present invention includes a first compartment containing at least one type of test substance and a first barcode nucleic acid corresponding to the test substance; a second compartment containing a cell or a derivative thereof and a second barcode nucleic acid-linked bead; and a third compartment, a fourth compartment and a fifth compartment each obtained by fusing the first compartment and the second compartment. Each of the compartments is a unit of section that can differentiate a combination of the components in a compartment from other compartments.

The form of the compartment of the present invention is not particularly limited, and may be, for example, a well, a droplet or a gel particle. More specific examples of the form include an aqueous droplet (e.g., an aqueous droplet in oil), an oil droplet, a gel particle of a hydrogel, a water-oil structure in which a plurality of non-mixed interfaces overlap, such as an emulsion, a vesicle with a monomolecular membrane or a double molecular membrane such as a micelle or liposome, and a well such as a multiwell plate.

In addition, the base material of the compartment is preferably an aqueous base material such as a hydrogel (for example, a material used as a scaffold in cell culture, including agarose such as low-melting agarose, collagen and alginic acid), more preferably low-melting agarose, from the viewpoint of measuring the effect of the test substance on cells. In addition, the compartment may contain a cell culture medium and saline.

The compartment of the present invention preferably has a physical barrier function at its peripheral edge part, from the viewpoint of clear distinction from other compartments. A suitable method for producing a compartment having the barrier function is, for example, a phase separation method. In the phase separation method, a compartment can be formed, for example, by mixing a cell and a bead with an aqueous base material to obtain an aqueous droplet, and subsequently suspending the aqueous droplet in a hydrophobic solvent.

### Cell or derivative thereof

The type and the form of a cell to be detected are not particularly limited as long as the effects of the present invention are not impaired, and a cell can be selected according to the object. The above cell contained in the compartment may be a derivative of a cell. Examples of the derivative include a homogenate of a cell, a content of a cell, a lysate of a cell, and a unit composed of cells (e.g., a cell cluster, a spheroid, an organoid).

The derivative of a cell (e.g., a homogenate, a content or a lysate of a cell) can be obtained by using a known method such as making a cell coexist with a cell lysis buffer or the like. A step of obtaining, from a cell, a derivative thereof may be performed by enclosing a cell lysis buffer or the like together when the first compartment and the second compartment are fused to form the third compartment, or the derivative may be produced in the second compartment by enclosing a cell lysis buffer together with the cell and the second barcode nucleic acid-linked bead.

The number of cells in the second and third compartments is not particularly limited as long as the effects of the present invention are not impaired, but is preferably one per compartment, from the viewpoint of single cell analysis. However, there is a high possibility that, for example, when one cell is contained in one compartment, the response is likely to vary. Therefore, the number of cells per compartment may be plural in order to perform more reliable response measurement, and the present invention also encompasses such an embodiment.

A linker capable of linking the surface of the cell or derivative thereof to the first barcode nucleic acid (for example, a linker having an oligonucleotide region and a lipid region (cholesterol, etc.)) may be bonded to the cell or derivative thereof, from the viewpoint of stable production of the first compartment together with the first barcode nucleic acid.

### Test substance

The test substance of the present invention is not particularly limited as long as it is a test substance to which response in a desired cell is examined, and examples thereof include compounds (e.g., drugs and medium components) and cells.

The number of types of test substances in the first and third compartments is not particularly limited as long as the effects of the present invention are not impaired, but is preferably one type per compartment, from the viewpoint of simplification or clarification of the cell response. However, for example, when the response of a cell to a plurality of types of test substances is examined, the number of types of test substances per compartment may be plural, and the present invention also encompasses such an embodiment.

### First barcode nucleic acid

The first barcode nucleic acid of the present invention is not particularly limited as long as it contains a barcode region corresponding to each test substance. For example, the nucleic acid is an RNA, a DNA, or a combination thereof.

Each first barcode nucleic acid of the present invention preferably includes a first common barcode region which is common in and corresponds to the same test substance and a first hybridize region hybridizable with the second barcode nucleic acid, which will be described later. The use of sequence information of the first common barcode region enables one-to-one correspondence to and discrimination of the same test substance in the first and third compartments. Thus, the sequence information of the first common barcode region can be used to identify the test substance in the third compartments.

### First bead

The first barcode nucleic acid may be a nucleic acid alone, or may be linked to the first bead (hereinafter also referred to as first barcode nucleic acid-linked bead) or present in the first bead. As used herein, the bead is not particularly limited as long as the effects of the present invention are not impaired, and is preferably a particle to which a barcode nucleic acid can be linked, a particle which can contain a barcode nucleic acid, or an organism which can contain a barcode nucleic acid. Moreover, the form of the bead is also not limited.

It is preferable that a plurality of the same first barcode nucleic acids are linked to each first bead, or that a plurality of the same first barcode nucleic acids be present per the first bead.

It is also preferable that only one type of first barcode nucleic acid is linked to or present in the first bead.

When the first bead is a particle, the material thereof is not particularly limited, and examples thereof include a semiconductor such as a quantum dot (semiconductor nanoparticle) made of a semiconductor material such as cadmium selenide (CdSe), zinc sulfide (ZnS), cadmium sulfide (CdS), zinc selenide (ZnSe), zinc oxide (ZnO), or silicon dioxide (SiO₂); an inorganic substance such as a heavy metal such as gold; a hydrogel such as acrylamide, agarose, collagen, alginic acid, cellulose, chitosan, hyaluronic acid, silicone hydrogel, or PEG-based; a resin such as polystyrene, polypropylene, or a hydrophilic vinyl polymer (e.g., Toyopearl HW-65S (Tosoh Corporation)); or chemically crosslinked products of these hydrogel materials; or a hydrophilic vinyl polymer to which PEG or a derivative thereof is bound. It is preferably a hydrogel, more preferably acrylamide or alginic acid. Such a first bead can also become a support when a cell is cultured.

When the first bead is an organism, the type and the form of the organism are not particularly limited as long as the effects of the present invention are not impaired, and an organism can be selected according to the object. Examples of the organism include a eukaryote or a prokaryote, or a cell thereof, and, for example, a microorganism, and specific examples thereof include a bacterium such as Escherichia coli or a fungus such as yeast. The organism preferably contains a plasmid having a first barcode nucleic acid, and more preferably is capable of amplifying a plasmid having a first barcode nucleic acid.

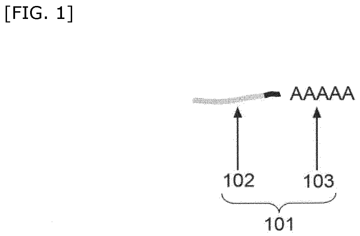

One embodiment of the first barcode nucleic acid of the present invention will be described in accordance with FIG. 1a, but the present invention is not particularly limited thereto.

FIG. 1 shows, as one embodiment of the first barcode nucleic acid, an embodiment in which the first barcode nucleic acid is a DNA. A first barcode nucleic acid 101 includes a first common barcode region 102 and a first hybridize region 103. Here, the first hybridize region 103 includes polyadenine.

### Method for producing first barcode nucleic acid

For the first barcode nucleic acid, a particular nucleic acid sequence is prepared by a solid-phase synthesis method or an enzyme synthesis method. When the barcode nucleic acid is an RNA, a DNA template, which becomes a complementary strand of a single-stranded barcode nucleic acid, is synthesized, and then synthesis may be performed by a linear amplification reaction using an RNA polymerase such as T7, which binds to a promoter sequence on the DNA template to synthesize an RNA including a single-stranded barcode region. When the barcode nucleic acid is a DNA, the barcode nucleic acid is not particularly limited as long as the effects of the present invention are not impaired, and may be synthesized and/or designed using, for example, a known sequence.

The first barcode nucleic acid-linked bead can be produced by the same production method as a method for producing a bead having a third barcode nucleic acid which will be described later.

### Second barcode nucleic acid-linked bead

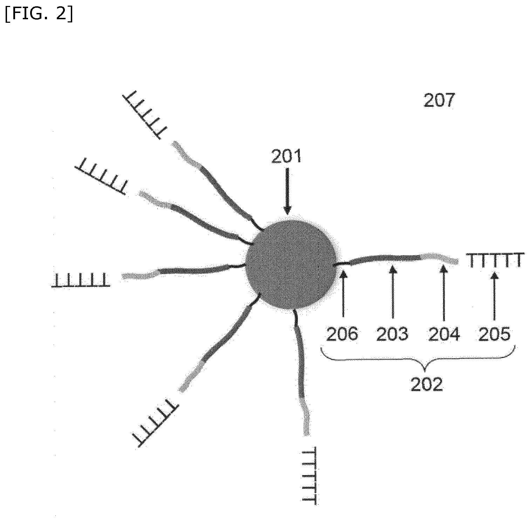

FIG. 2 shows one embodiment in which a second bead linked to a plurality of second barcode nucleic acids hybridizable with a genome-related nucleic acid and a first barcode nucleic acid (hereinafter also referred to as second barcode nucleic acid-linked bead).

FIG. 2 shows a second barcode nucleic acid-linked bead 207 in which a second barcode nucleic acid 202 is linked to a second bead 201. The second barcode nucleic acid 202 contains a second common barcode region 203, a second unique barcode region 204, and a second hybridize region 205. The second barcode nucleic acid 202 contains a PCR primer region 206, the second common barcode region 203, the second unique barcode region 204, and the second hybridize region 205 in this order from the second bead side. The above second hybridize region 205 is polythymine. Since the second barcode nucleic acid 202 contains the second unique barcode region 204, each second barcode nucleic acid has a different sequence. Therefore, the plurality of second barcode nucleic acids linked to the second bead preferably becomes a plurality of types of second barcode nucleic acids.

The second bead is preferably linked to 1,000 to 100,000 second barcode nucleic acids in terms of the fact that it can be hybridized with many genome-related nucleic acids.

### Second bead

As the material for the second bead of the present invention, the same material as for the first bead particle can be used.

The material for the second bead of the present invention is preferably a hydrogel or a resin, more preferably acrylamide, polystyrene, a hydrophilic vinyl polymer, or a hydrophilic vinyl polymer to which PEG or a derivative thereof is bound.

### Second barcode nucleic acid

The second barcode nucleic acid of the present invention is not particularly limited as long as it contains a barcode region, and, for example, the nucleic acid is an RNA, a DNA, or a combination thereof. The second barcode nucleic acid can be directly or indirectly linked to the second bead.

The second barcode nucleic acid of the present invention preferably contains a second common barcode region which is in common with each other, a second unique barcode region which can be clearly distinguished from each other, and a second hybridize region. Here, sequence information of the above second common barcode region can be used as an index for identifying a cell existing together with the second barcode nucleic acid in a compartment. Furthermore, sequence information of the above second unique barcode region can be used as an index for identifying a genome-related nucleic acid. The above second hybridize region can be hybridized with each of the genome-related nucleic acid, the first barcode nucleic acid, the third barcode nucleic acid, and a fourth barcode nucleic acid which will be described later. Therefore, the second hybridize region preferably contains polythymine or a nucleic acid complementary to the genome-related nucleic acid.

As described above, since the genome-related nucleic acid is associated with sequence information of the above second common barcode region, it is possible to identify a cell from which the genome-related nucleic acid is derived. Furthermore, since the above second barcode nucleic acid is hybridized with the first barcode nucleic acid, sequence information of the first common barcode region such as sequence information of the first barcode nucleic acid is also associated with sequence information of the above second common barcode region. The sequence information of the first common barcode region corresponds to each test substance as described above.

Furthermore, since the above second barcode nucleic acid is hybridized with the third barcode nucleic acid, sequence information of the third common barcode region or the like of the bead having a third barcode nucleic acid is also associated with sequence information of the above second common barcode region. Sequence information of the third common barcode region is associated with imaging information of each third bead. Nondestructive measurement information of the cell is also associated with imaging information of each third bead. Therefore, one-to-one correspondence is possible between genome-related nucleic acid information and nondestructive measurement information of the cell.

### Method for producing second barcode nucleic acid-linked bead

A method for producing a plurality of types of second barcode nucleic acid-linked beads can be performed by a known method. For example, the second barcode nucleic acid-linked beads can be produced by the method described in E. Z. Macosko et al., Highly Parallel Genome-wide Expression Profiling of Individual Cells Using Nanoliter Droplets. Cell. 161, 1202-1214 (2015) or Gierahn, T. M et al., Seq-Well: A Portable, Low-Cost Platform for High-Throughput Single-Cell RNA-Seq of Low-Input Samples;Nat Methods. 14, 395-398 (2017).

The above method is briefly described. The second common barcode region in the second barcode nucleic acid can be produced by split-and-pool synthesis. For example, it can be produced by performing n rounds of the split-and-pool synthesis. Each round consists of i) a step of splitting a bead population into four pieces, ii) a step of synthesizing any of A, G, C, and T for each bead population, and iii) a step of combining and pooling the four bead populations. The number of rounds n can be appropriately set according to the length of a barcode sequence to be produced. For example, n = 6 to 40 is exemplified.

After the above second common barcode is produced, a second unique barcode region is synthesized. For all beads linked to the second common barcode region, synthesis in the presence of all bases of A, T, G, and C is performed for m rounds. The number of rounds m can be appropriately set according to the length of a barcode sequence to be produced. For example, m = 3 to 15 is exemplified.

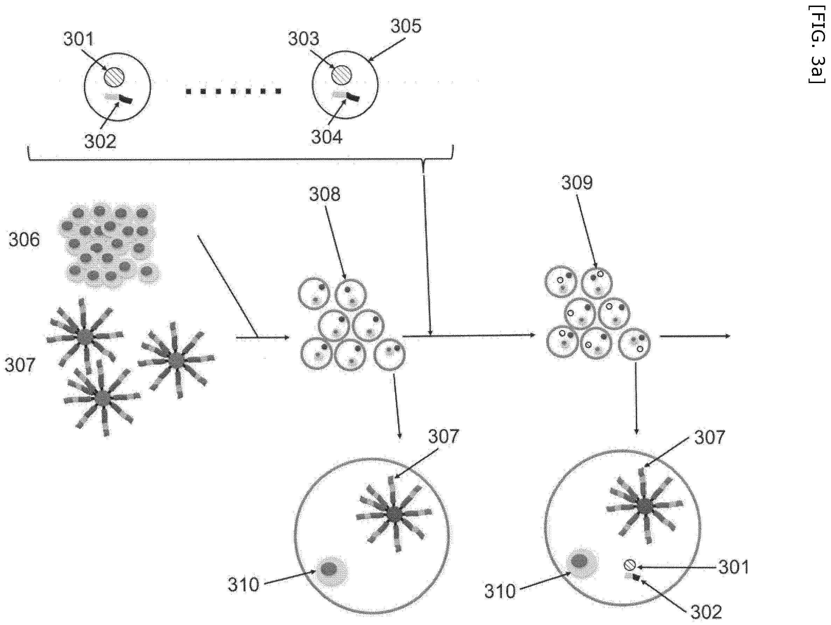

One embodiment of the step of preparing first to third compartments of the present invention will be described in accordance with FIG. 3a, but the present invention is not particularly limited thereto.

First, n types of test substances, i.e., a test substance 1 301 to a test substance n 303, and first barcode nucleic acids 302 and 304 corresponding to the test substance 1 301 and the test substance n 303, respectively, are prepared. Then, first compartments 305 each containing the first barcode nucleic acid corresponding to one test substance are prepared. In FIG. 3a, the first barcode nucleic acid is a DNA, and includes a first common barcode region and polyadenine.

Moreover, a cell group 306 to be detected such as a tissue or a plurality of cells, and a plurality of second barcode nucleic acid-linked beads 307 are each prepared. The second barcode nucleic acid is a DNA, and contains a PCR primer region, a second common barcode region, a second unique barcode region, and polythymine.

In FIG. 3a, a first component is formed so as to contain one type of test substance and one type of barcode nucleic acid corresponding thereto. By the first compartment formation step, one type of test substance and the barcode nucleic acid corresponding thereto are associated with each other. Specifically, the first compartment is formed by mixing one type of test substance and one type of barcode nucleic acid corresponding thereto in a well or the like.

On the other hand, the cell group 306 and the plurality of second barcode nucleic acid-linked beads 307 are partitioned to obtain a plurality of types of second compartments 308. By the partitioning, a combination of a single cell 310 in the above cell group 306 and the second barcode nucleic acid-linked bead 307 are partitioned into the plurality of second compartments 308.

A partitioning method will be described later.

The number of the above second barcode nucleic acid-linked beads per compartment is not particularly limited, but is preferably one per compartment from the viewpoint of discrimination of genome-related information derived from the same single cell or genome-related information of a plurality of cells in the same compartment.

Then, one first compartment 305 and one second compartment 308 are fused to form a plurality of types of third compartments 309. The cell or derivative thereof coexists with the test substance in each third compartment.

A known method can be used as the method for forming the third compartments 309 by fusing one first compartment 305 and one second compartment 308. For example, the methods described in B.L. Wang et al., Nat Biotechnol. 32(5), 473-8 (2014 May); B. Demaree et al., J Vis Exp. (135), 57598 (2018); L. Xu et al., Lab Chip. 12(20), 3936-42 (2012); and L. Mazutis et al., Lab Chip. 9(20), 2902-8 (2009) are exemplified.

Furthermore, during partitioning, reagents necessary for the subsequent steps, for example, PCR reagents such as PCR Reaction Mix may be enclosed simultaneously.

### Step of culturing cell in coexistence with test substance

According to need, the cell or derivative thereof may be cultured in a state where it coexists with the test substance in the third compartments. The culture includes, for example, retaining the third compartments for a desired culture time and at a desired culture temperature. In the retention of the third compartments, the third compartments may be moved to and retained in a reservoir capable of retaining the plurality of types of third compartments. The above step can be performed by a known technique. For example, the step can be performed by the method described in J. J. Agresti et al., Proc Natl Acad Sci U S A., 107(9), 4004-9 (2010); A. Abbaspourrad et al., Sci Rep., 5, 12756 (2015); or B. L. Wang et al., Nat Biotechnol., 32(5), 473-8 (2014).

The culture time and the culture temperature can be set to such a culture time and a culture temperature as to enable evaluation of the response of the cell to the test substance. The culture time is, for example, 0 hours or more and 14 days or less, preferably 2 hours or more and 2 days or less. The culture temperature is, for example, 0°C or higher and 100°C or lower, preferably 20°C or higher and 40°C or lower.

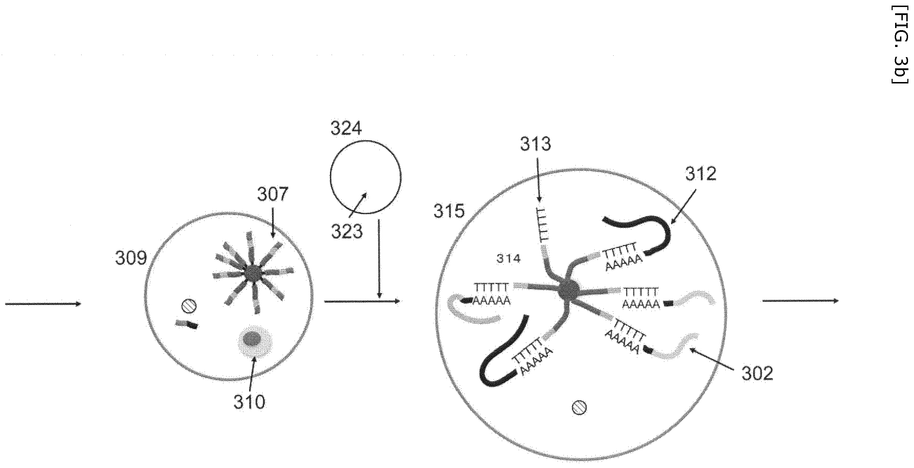

When the cell or derivative thereof is cultured in the coexistence with the test substance in the third compartments, the response of the cell to the test substance takes place during cell culture. Therefore, a step of adding a cell lysis buffer is preferably included after the culture. The above step can be performed by a known technique. For example, as shown in FIG. 3b, a fourth compartment 324 containing an aqueous solvent 323 which contains a cell lysis buffer and the third compartment 309 may be fused to form a fifth compartment 315. Examples of the fusion method include the methods described in B.L. Wang et al., Nat Biotechnol. 32(5), 473-8 (2014 May);B. Demaree et al., J Vis Exp. (135), 57598 (2018); L. Xu et al., Lab Chip. 12(20), 3936-42 (2012); and L. Mazutis et al., Lab Chip. 9(20), 2902-8 (2009).

### Step of obtaining hybridized complex

A step of obtaining a hybridized complex includes a step of hybridizing each of the genome-related nucleic acid and the first barcode nucleic acid with the second barcode nucleic acid to obtain a hybridized complex.

The above step can be performed by a known technique. For example, the step can be performed by the method described in E. Z. Macosko et al., Highly Parallel Genome-wide Expression Profiling of Individual Cells Using Nanoliter Droplets. Cell. 161, 1202-1214 (2015) or Zheng GX et al., Massively parallel digital transcriptional profiling of single cells. Nat Commun. 6; 8:14049 (2017).

One embodiment of the step of obtaining a hybridized complex will be described in accordance with FIG. 3b, without any particular limitation.

As described above, the cell is lysed following the step of forming a plurality of types of third compartments, and, according to need, the step of culturing a cell in the coexistence with a test substance, and the step of adding a cell lysis buffer. Then, in the compartment, each of a cell-derived mRNA 312 and the first barcode nucleic acid 302 is hybridized with the second barcode nucleic acid 313 to obtain a hybridized complex 314. Then, the droplet is destroyed.

### Step of producing amplified product derived from hybridized complex

A step of producing an amplified product derived from the hybridized complex includes a step of producing an amplified product derived from the hybridized complex obtained in the above step of obtaining a hybridized complex.

The above step can be performed by a known technique. For example, the step can be performed by the method described in E. Z. Macosko et al., Highly Parallel Genome-wide Expression Profiling of Individual Cells Using Nanoliter Droplets. Cell. 161, 1202-1214 (2015) or Zheng GX et al., Massively parallel digital transcriptional profiling of single cells. Nat Commun. 6; 8:14049 (2017).

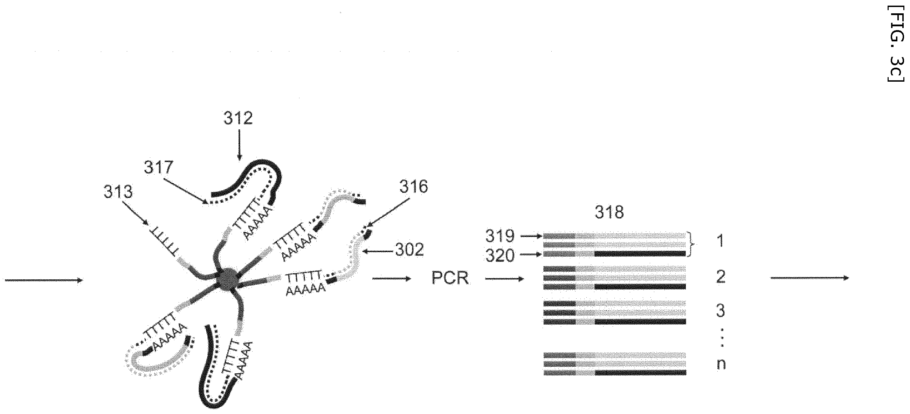

One embodiment of the step of producing an amplified product derived from the hybridized complex will be described in accordance with FIG. 3c, without any particular limitation.

Complementary strand DNA synthesis and reverse transcription reaction are performed on the hybridized complex obtained in the above step of obtaining a hybridized complex. By this synthesis and reverse transcription reaction, a cDNA 317 for the cell-derived mRNA 312 is synthesized, and a complementary strand DNA 316 for the first barcode nucleic acid 302 is synthesized. Then, template switching may be performed.

Then, a PCR reaction is performed. This PCR reaction produces two types of amplified products 318, a first amplified product 319 derived from the hybridized complex of the first barcode nucleic acid 302 with the second barcode nucleic acid 313 and a second amplified product 320 derived from the hybridized complex of the cell-derived mRNA 312 with the second barcode nucleic acid 313. When the genome-related nucleic acid is a DNA, an extension PCR method can be performed as the above PCR reaction.

Then, based on the thus-obtained amplified product, a library of amplified products including the first amplified product and the second amplified product derived from each of the third compartments containing the test substances 1 to n, respectively, is prepared.

### Step of detecting genome-related information in cell after coexistence with test substance

A step of detecting genome-related information in a cell after coexistence with a test substance includes a step of identifying a test substance coexisting with a cell and detecting genome-related information of the cell, using, as an index, an expression pattern of an amplified product obtained by the step of producing amplified product derived from hybridized complex. Examples of the above expression pattern of the amplified product include sequence information of the amplified product such as sequence information of the first barcode nucleic acid (e.g., sequence information of the first common barcode region) and sequence information of the second barcode nucleic acid (e.g., sequence information of the second common barcode region and sequence information of the second unique barcode region), in the sequence information, which are obtained by sequencing.

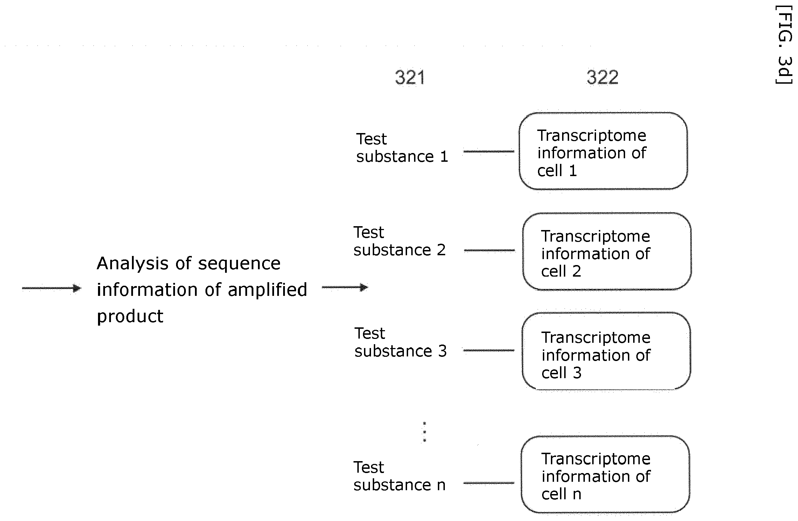

One embodiment of the step of detecting genome-related information in a cell after coexistence with a test substance will be described in accordance with FIG. 3d, without any particular limitation.

The sequence of the amplified products (the first amplified product and the second amplified product) obtained in the above step of producing an amplified product derived from the hybridized complex is determined by a sequencer, and sequence information of the amplified products is analyzed. In the analysis of the second amplified product, a cell from which each amplified product is derived is assigned using sequence information of the second common barcode region as an index. Since each mRNA molecule can be discriminated by sequence information of the second unique barcode region, information such as the sequence of an mRNA for each cell and the expression amount thereof can be obtained using the sequence information as an index. Based on the information obtained by the above analysis of the second amplified product, transcriptome information 322 for each cell can be obtained.

The test substance 321 coexisting with the above cell is identified. The first barcode nucleic acid corresponds to the test substance 321, as described above. Here, in the identification, the test substance 321 coexisting with the cell can be assigned to each first amplified product based on sequence information of the first common barcode region of the first barcode nucleic acid.

Next, the test substance 321 coexisting with the cell is matched to transcriptome information 322. Therefore, one-to-one linking is possible between genome-related information of the cell and the coexisting test substance in each third compartment.

Thus, by detecting genome-related information such as transcriptome information of the cell or derivative thereof coexisting with at least one type of test substance, the response of the cell to the coexisting test substance can be evaluated.

### Method for detecting nondestructive measurement information and genome-related information of cell coexisting with at least one type of test substance

According to a second embodiment of the present invention, the present invention further encompasses, in addition to the detection method according to the first embodiment, a method for detecting nondestructive measurement information and genome-related information of a cell coexisting with at least one type of test substance integrally.

The method for detecting genome-related information of a cell or a derivative thereof coexisting with at least one type of test substance is characterized that:
the second compartment further contains a bead having a third barcode nucleic acid,
wherein the bead having a third barcode nucleic acid is:
   a particle cleavably linked to a third barcode nucleic acid corresponding to each imaging information or
   an organism containing a third barcode nucleic acid corresponding to each imaging information,
imaging information of the bead having a third barcode nucleic acid in the third compartment can be clearly distinguished from each other, and
the method further including:
   detecting both nondestructive measurement information of the cell and imaging information of the bead having a third barcode nucleic acid and associating the nondestructive measurement information of the cell with the imaging information of the bead having a third barcode nucleic acid;
   cleaving or taking out the third barcode nucleic acid from the associated bead having the third barcode nucleic acid, and hybridizing each of the genome-related nucleic acid and the third barcode nucleic acid with the second barcode nucleic acid to obtain a hybridized complex;
   producing an amplified product derived from the hybridized complex; and
   integrally detecting nondestructive measurement information and genome-related information of the cell using an expression pattern of the amplified product as an index.

Any of the above embodiments of compartments, cell or derivative thereof, test substance, first barcode nucleic acid and production method therefor, first beads, second barcode nucleic acid-linked bead and production method therefor, second bead, and second barcode nucleic acid in the above second embodiment can follow the descriptions in the above first embodiment.

### Bead having third barcode nucleic acid

A third bead, which is used in the bead having a third barcode nucleic acid of the present invention, is used as an index for identifying non-destructive measurement information of a cell or a derivative thereof in each compartment, based on imaging information thereof. Therefore, the third bead of the present invention can be partitioned into a compartment together with a cell or a derivative thereof, and can be a bead whose imaging information can be measured. In addition, the bead partitioned into each compartment functions as an index for identifying non-destructive measurement information of a cell or a derivative thereof in the compartment, and therefore preferably has imaging information that can be clearly distinguished from each other.

The above imaging information of the third bead is not particularly limited as long as imaging information of the third bead in each compartment can be clearly distinguished from each other. The imaging information may be imaging information possessed by the bead itself or imaging information imparted by labeling. Here, "imaging" encompasses a method capable of separating and measuring the measurement information of a test target such as a bead, which temporally overlaps based on spatial information. Measurement information of a test target obtained by the above imaging is referred to as imaging information. Examples of the above imaging information include at least one piece of measurement information selected from color, fluorescence, size, shape, electromagnetic wave, transmission, phase, scattering, reflection, coherent Raman, infrared spectroscopy, Raman spectroscopy, absorption spectrum, and the number of third beads. The imaging information is preferably infrared spectroscopy imaging, Raman spectroscopy imaging, color imaging, and fluorescence imaging.

The fluorescence can be obtained by organic fluorescent molecules, organism-derived fluorescent molecules, quantum dots, inorganic substances such as heavy metals, or a combination thereof.

Measurement information such as transmission, phase, scattering, and reflection can be obtained by organic substances or inorganic substances having a different refractive index or color depending on their concentration or a combination thereof. These types of information can be obtained by a method such as bright field observation method.

Absorption spectrum and Raman can be obtained by organic molecules or inorganic substances having a different absorption waveband of absorption and Raman scattering spectra (molecular footprint) or a combination thereof, and examples thereof include alkyne-based compounds having a wavelength range which does not overlap with a cell signal.

Coherent Raman can be measured by, for example, the coherent anti-Stokes Raman scattering (CARS) method or the stimulated Raman scattering (SRS) method.

The size, color, and shape of a bead also become imaging information of the third bead, and these size, color, and shape can be diverse by forming the bead by, for example, flow lithography. These types of information can be obtained by a method such as bright field observation method.

Since imaging information of a bead is spatially separated from a cell, it can be separated without interfering with nondestructive measurement information of the cell.

The third bead is not particularly limited as long as it is a particle to which a barcode nucleic acid can be linked or an organism which can contain a barcode nucleic acid, and its shape is not limited.

When the third bead is a particle, the same material as for the first bead can be used. The material for the third bead is preferably a hydrogel, more preferably acrylamide or alginic acid.

When the third bead is an organism, the same organism as for the first bead can be used. Examples of the organism include a bacterium such as Escherichia coli and a fungus such as yeast. The organism is preferably capable of amplifying a plasmid having a third barcode nucleic acid.

The number of the above third beads per compartment is not particularly limited, and may be 1, but is preferably plural, more preferably 2 to 100, and still more preferably 2, 3, 4, 5, 6, 7, 8, 9, and 10 in order to increase the number of types of compartments which can be clearly distinguished from each other. Enclosing a plurality of third beads into the compartment at one time enables rapid increase in the number of variations of a combination of imaging information of the third beads in the compartment by a combination of imaging information of a few types of third beads, and enables clear distinction of a large amount of compartments from each other. Here, third beads having the same imaging information also exist, but it is preferable that a plurality of third beads having different types of imaging information exist in the compartment. For example, an example in which size of the bead, color of fluorescence, and concentration of fluorescence are selected as the imaging information of the third bead will be shown below. It is assumed that the size of the bead includes 3 types (3, 7, and 11 µm), the color of the fluorescent dye includes 3 colors (blue, green, and red), and the intensity level according to the concentration of the fluorescent dye includes 6 types (0, 1, 2, 3, 4, and 5). In this case, the type of the third bead will be (intensity level^{size type} - 1) × size type = (6³ - 1) × 3 = 645.

Here, when three third beads exist in the compartment, the types of each combination of the third beads are ₆₄₅C₃ > 10⁷ types, and thus great many types of combinations can be obtained.

### Nondestructive measurement information of cell

The above nondestructive measurement information of a cell is not particularly limited as long as the feature of the cell can be recognized, examples thereof include imaging information, morphological information obtained from the cell, measurement information of a physical wave (e.g., sound, ultrasonic wave) obtained from the cell, and measurement information of an electromagnetic wave (e.g., light, terahertz) obtained from the cell. Here, the above imaging information can be obtained in the same manner as for imaging information of the third bead. Examples of the above nondestructive measurement information include imaging information based on measurement information such as color, fluorescence, size, shape, electromagnetic wave, transmission, phase, scattering, reflection, coherent Raman, infrared spectroscopy, Raman, or absorption spectrum, or morphological information of a cell such as nucleus, size of the cytoplasm, coarseness and fineness of the cytoskeleton, feature amount of the internal structure, uniformity of the membrane, fluorescence intensity of each structure, molecular localization, or positional relationship of the molecule or the subject to be observed, and the nondestructive measurement information is preferably morphological information obtained from the cell.

### Third bead which is cleavably linked to third barcode nucleic acid and which has imaging information that can be clearly distinguished from each other

The bead having a third barcode nucleic acid in the present invention is preferably a particle cleavably linked to a third barcode nucleic acid corresponding to each imaging information or an organism containing a third barcode nucleic acid corresponding to each imaging information.

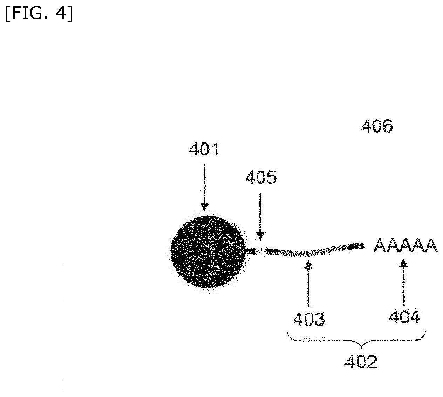

FIG. 4 shows an embodiment in which the third barcode nucleic acid is an RNA as one embodiment of a third bead 406 which is cleavably linked to a third barcode nucleic acid and which has imaging information that can be clearly distinguished from each other (hereinafter also referred to as third barcode nucleic acid-linked bead).

A third barcode nucleic acid 402 is linked to a third bead 401 having imaging information that can be clearly distinguished from each other. The third barcode nucleic acid 402 contains a third common barcode region 403 and a third hybridize region 404. The third barcode nucleic acid 402 contains the third common barcode region 403 and the third hybridize region 404 in this order from the third bead side. Here, the third hybridize region 404 includes polyadenine. Furthermore, the third barcode nucleic acid 402 is cleavably linked to the bead 401 via a cleavable linker 405.

### Organism containing third barcode nucleic acid

The third bead of the present invention may be an organism containing a third barcode nucleic acid and having imaging information that can be clearly distinguished from each other (hereinafter also referred to as organism containing a third barcode nucleic acid). The organism preferably contains a plasmid having a third barcode nucleic acid. Imaging information in the organism is not particularly limited as long as it is imaging information of the present invention, but it is preferably the number of organisms or fluorescence. Examples of the fluorescence include not only a spectrum of each color but also brightness information thereof. Furthermore, fluorescence is preferably obtained from a fluorescent protein expressed from a fluorescent protein gene existing in a plasmid. Therefore, the organism of the present invention preferably contains, for example, a plasmid having a third barcode nucleic acid and a fluorescent protein gene. Here, the third barcode nucleic acid contains a third common barcode region (e.g., a common barcode region common for each fluorescent protein) and a third hybridize region. The third hybridize region includes polyadenine. Furthermore, the third barcode nucleic acid may contain a third unique barcode region. The third unique barcode region enables clear distinction of each clone of an organism. Therefore, the constitution of a plasmid in the organism of the present invention is, for example, a fluorescent protein gene region, a third unique barcode region, a third common barcode region, and a third hybridize region in this order.

### Third barcode nucleic acid

The third barcode nucleic acid of the present invention is not particularly limited as long as it contains a barcode region corresponding to each imaging information, and for example, the nucleic acid is an RNA, a DNA, or a combination thereof.

Each third barcode nucleic acid of the present invention preferably includes a third common barcode region which is common in the third bead corresponding to same imaging information and a third hybridize region hybridizable with the second barcode nucleic acid. By using sequence information of the third common barcode region, one-to-one correspondence is possible to imaging information of the third bead having the same imaging information in each compartment. Therefore, association can make an index of specifying nondestructive measurement information of a cell existing in the same compartment.

It is preferable that a plurality of the same third barcode nucleic acids are linked to one third bead.

It is preferable that one type of the third barcode nucleic acid is linked to the third bead of the present invention.

The third barcode nucleic acid can be directly or indirectly linked to the third bead. The third barcode nucleic acid is preferably cleavably linked to the third bead, and, for example, can be linked via a cleavable linker. In the present invention, examples of the cleavable linker include a chemically cleavable linker, a photocleavable linker such as UV-cleavable linker, a thermologically cleavable linker, an enzymatically cleavable linker or the like. By using the above linker, it is shown that the linked nucleic acid is cleaved from the bead, and can be separated or released. Examples of such a linker include PC-biotin, iSpPC or the like as a photocleavable linker or a disulfide bond or the like as a chemically cleavable linker.

As another preferred embodiment of the third bead of the present invention, the third bead may further contain a third unique barcode region and a primer region each of which can be clearly distinguished from each other. As further another preferred embodiment of the third bead of the present invention, the third bead may have an acrylamide moiety such as an acrylic phosphoramidite moiety (Acrydite (trademark)) via a cleavable linker.

### Method for producing third bead which is cleavably linked to third barcode nucleic acid and which has imaging information that can be clearly distinguished from each other

A method for producing a third bead linked to a third barcode nucleic acid can be performed according to a known method. For example, the third bead can be produced according to the method mentioned in A. M. Klein et al., Droplet Barcoding for Single-Cell Transcriptomics Applied to Embryonic Stem Cells. Cell. 161, 1187-1201 (2015).

As an example of the above method, by microfluidic emulsion technology, an aqueous solution of third barcode nucleic acid-containing acrylamide:bisacrylamide is made to be an acrylamide polymer in an organic solvent layer, and this is used as the third bead. Here, the bead-linked side of a cleavable linker bound to a third barcode nucleic acid can be modified by an acrylamide moiety such as an acrylic phosphoramidite moiety (Acrydite (trademark)). By the above modification, the third barcode nucleic acid is also polymerized into an acrylamide polymer during polymerization of acrylamide. Before this emulsification, for example, by dissolving a fluorescence-labeled acrylamide monomer in the above aqueous solution, it is possible to produce third beads having various fluorescence intensities of each color. Specifically, the third bead can be produced by a droplet production method using a flow focusing device or microfluidic techniques such as a microwell. The size of the bead can be controlled by changing the fluidic conditions for the flow focusing device and by changing the size of each chamber for the microwell. After polymerization, the third bead thus obtained is taken out from the droplet and washed several times, and this is used as the third bead.

The third bead linked to a third barcode nucleic acid can be produced by a known method. For example, the third bead can be produced by the method mentioned in JP 2009-513948 T or JP 2017-506877 T.

An example of the above method will be described. First, for the third barcode nucleic acid, a particular nucleic acid sequence is prepared by a solid-phase synthesis method or an enzyme synthesis method. Then, it is bound to the third bead via a cleavable linker. When the barcode nucleic acid is an RNA, a DNA template, which becomes a complementary strand of a single-stranded barcode nucleic acid, is synthesized, and then an RNA may be synthesized by a linear amplification reaction using an RNA polymerase such as T7, which binds to a promoter sequence on the DNA template to synthesize an RNA including a single-stranded barcode region.

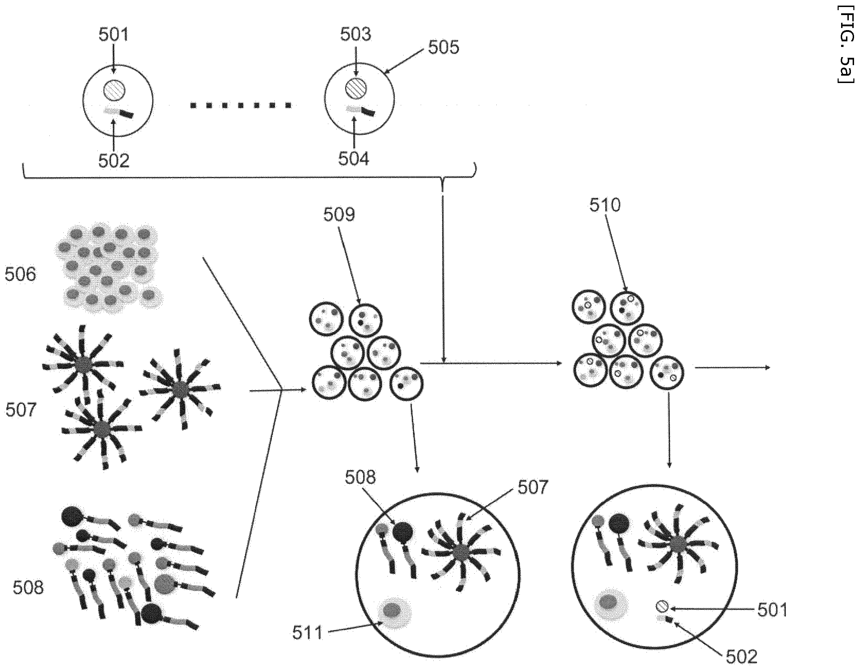

Another embodiment of the step of preparing first to third compartments in the second embodiment of the present invention will be described in accordance with FIG. 5a, but the present invention is not particularly limited thereto.

First, n types of test substances, i.e., a test substance 1 501 to a test substance n 503, and first barcode nucleic acids 502 and 504 corresponding to the test substance 1 501 and the test substance n 503, respectively, are prepared. Then, first compartments 505 each containing the first barcode nucleic acid corresponding to one test substance are prepared. In FIG. 5a, the first barcode nucleic acid is a DNA, and includes a first common barcode region and polyadenine.

Moreover, a cell group 506 to be detected such as a tissue or a plurality of cells, a plurality of types of second barcode nucleic acid-linked beads 507, and a bead 508 having a plurality of types of third barcode nucleic acids are each prepared. In FIG. 5a, the third barcode nucleic acid is an RNA, contains a third common barcode region and polyadenine, and is linked to a third bead via a UV-cleavable linker. The second barcode nucleic acid is an DNA, and contains a PCR primer region, a second common barcode region, a second unique barcode region, and polythymine.

In FIG. 5a, a first component is formed so as to contain one type of test substance and one type of barcode nucleic acid corresponding thereto. Specifically, the first compartment is formed by mixing one type of test substance and one type of barcode nucleic acid corresponding thereto in a well or the like.

On the other hand, the cell group 506, the plurality of types of second barcode nucleic acid-linked beads 507, and the bead 508 having a plurality of types of third barcode nucleic acids are partitioned to obtain a plurality of types of second compartments 509. By the above partitioning, a combination of a single cell 511 in the above cell group 506, the second barcode nucleic acid-linked beads 507, and the bead 508 having a plurality of types of third barcode nucleic acids are partitioned into the plurality of types of second compartments 509.

A partitioning method will be described later.

The number of the above second barcode nucleic acid-linked beads per compartment is not particularly limited, but is preferably one per compartment from the viewpoint of discrimination of genome-related information derived from the same single cell or genome-related information of a plurality of cells in the same compartment.

Then, one first compartment 505 and one second compartment 509 are fused to form a plurality of types of third compartments 510. The cell or derivative thereof coexists with the test substance in each third compartment.

A known method can be used as the method for forming the third compartments 510 by fusing one first compartment 505 and one second compartment 509. For example, the methods described in B.L. Wang et al., Nat Biotechnol. 32(5), 473-8 (2014 May); B. Demaree et al., J Vis Exp. (135), 57598 (2018); L. Xu et al., Lab Chip. 12(20), 3936-42 (2012); and L. Mazutis et al., Lab Chip. 9(20), 2902-8 (2009) are exemplified.

Here, the positional relationship between the cell and the third bead in each compartment is not particularly limited as long as obtaining of nondestructive measurement information of the cell and imaging information of the third bead is not impaired, and it can be appropriately set according to the type, size, and nature of the cell and the third bead. In other words, in the present invention, as long as imaging information of both the cell and the third bead can be obtained, both of them may coexist in a state in which the third bead and the cell are or are not in contact with each other in the same compartment, and the third bead may be introduced into the inside of the cell. Suitable examples of an embodiment in which the third bead and the cell are in contact with each other include an embodiment in which the cell surface and the third bead are directly adhered to each other or the third bead is attached on the cell by using a commercially available cell membrane modifier, or the like. Suitable examples of an embodiment in which the third bead and the cell are not in contact with each other include an embodiment in which the third bead exists without being joined to the cell within the same compartment, or an embodiment in which, by conjugating a subcompartment encompassing the third bead but no cell to the cell, the subcompartment is attached to the cell, or the like. Suitable examples of an embodiment in which the third bead is introduced into the inside of the cell include an embodiment in which the third bead is ingested by the cell, or the like.

Furthermore, during partitioning, reagents necessary for the subsequent steps, for example, PCR reagents such as PCR Reaction Mix may be enclosed simultaneously.

### Step of associating nondestructive measurement information of cell with imaging information of bead

A step of associating nondestructive measurement information of a cell with imaging information of a bead of the present invention includes a step of detecting both nondestructive measurement information of the cell and imaging information of the bead having a third barcode nucleic acid and associating the nondestructive measurement information of the cell with the imaging information of the bead having a third barcode nucleic acid.

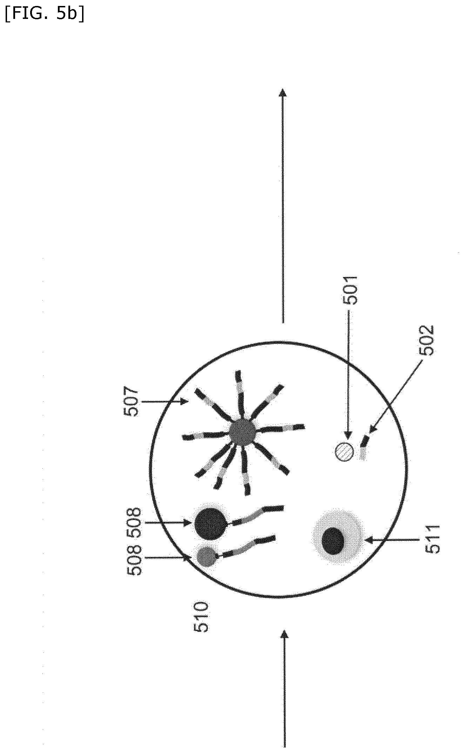

One embodiment of the step of associating nondestructive measurement information according to second embodiment of the present invention will be described in accordance with FIG. 5b, but the present invention is not particularly limited thereto.

Both of nondestructive measurement information of the single cell 511 in the third compartment 510 and imaging information of the bead 508 having a third barcode nucleic acid are measured. Furthermore, based on the obtained measurement results, the nondestructive measurement information of the single cell 511 in the third compartment 510 is associated with the imaging information of the bead 508 having a third barcode nucleic acid.

Examples of the above method for detecting or measuring both of nondestructive measurement information of the cell and imaging information of the third bead include flow cytometry (e.g., an imaging flow cytometry method which observes a compartment flowing in a flow pass), and a microscopic measurement (e.g., a method for observing a compartment in a microwell using a general light microscope).

According to another embodiment of the present invention, since the third compartment 510 can be identified by imaging information of a bead, for example, after obtainment of imaging information and nondestructive measurement information of a cell before coexistence of a test substance and the cell, it is also possible to measure nondestructive measurement information of the cell and the like again after the elapse of a predetermined time, e.g., after 6 hours to 2 days has passed since the test substance and the cell coexisted. By repeating such detection, nondestructive measurement information of the cell can be monitored.

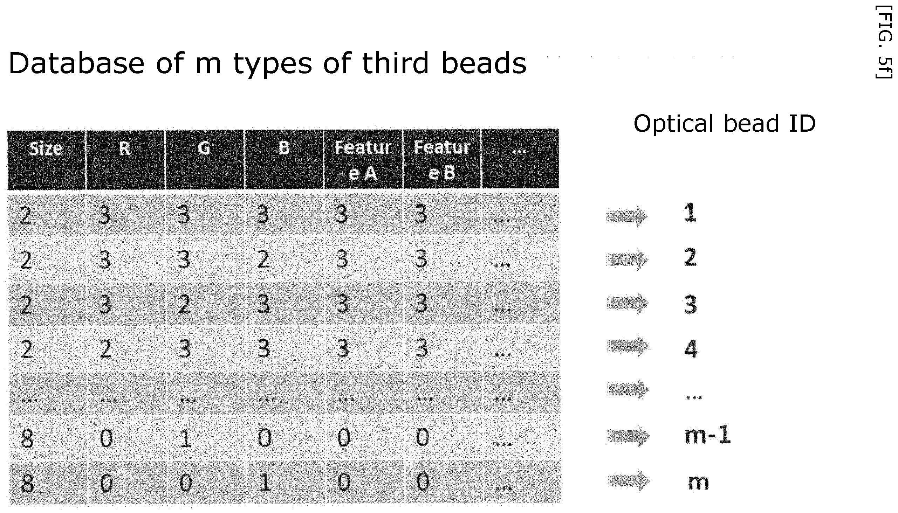

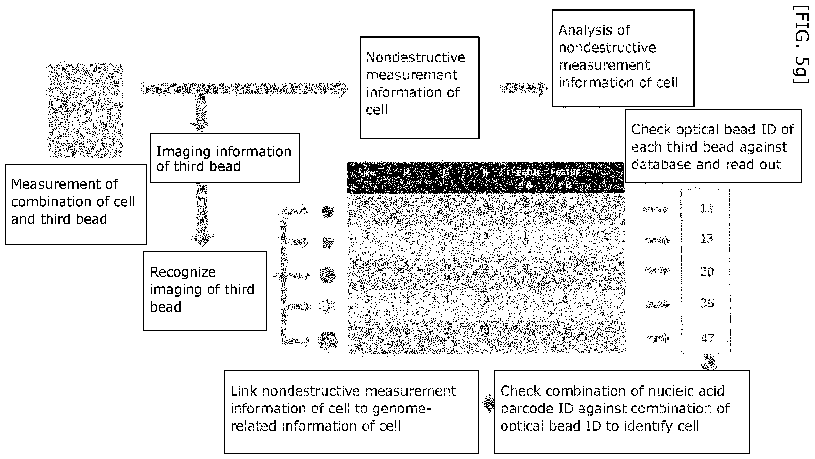

In FIG. 5f and FIG. 5g, an analytical scheme of imaging information will be more specifically described as an example.

FIG. 5f is a schematic diagram of a database of the third bead when imaging information of m types (ID) of the third beads produced by controlling the size, RGB, fluorescent brightness, and other optical properties is used as an index. In FIG. 5f, imaging information of the third bead is measured, and an optical bead ID is linked to the imaging information of the third bead thus obtained.

Furthermore, in FIG. 5g, a combination of a cell and a third bead is measured, and nondestructive measurement information of the cell is associated with imaging information of the third bead. First, the optical barcode ID of each third bead is read (here, optical calibration is performed). To each third bead, a nucleic acid barcode ID is imparted based on the third common barcode region of the third barcode nucleic acid linked to the third bead. Therefore, a combination of the above nucleic acid barcode ID of the bead having a third barcode nucleic acid is checked against a combination of the above optical bead ID to identify the cell. Subsequently, nondestructive measurement information of the cell can be linked to genome-related information of the cell.

The step of detecting and associating nondestructive measurement information of a cell and imaging information of the third bead of the present invention may be performed not only at the above third compartment but also before preparation of the third compartment and/or in the second compartment. Specifically, both non-destructive measurement information of a cell and imaging information of a bead having a third barcode nucleic acid can be detected before preparation of the second or third compartment before coexistence of a test substance and the cell, and/or after the elapse of a predetermined time after coexistence of a test substance and the cell.

Here, the positional relationship between the cell and the third bead in the embodiment in which both nondestructive measurement information of a cell and imaging information of the third bead are detected and associated before preparation of the second compartment is the same as the above positional relationship between the cell and the third bead in the second or third compartment, and is not particularly limited as long as obtainment of the nondestructive measurement information of the cell and the imaging information of the third bead is not impaired. The positional relationship can be appropriately set according to the types, sizes, and natures of the cell and the third bead. In other words, in the present invention, as long as both the nondestructive measurement information of the cell and the imaging information of the third bead can be obtained, both of them may coexist in a state in which the third bead and the cell are or are not in contact with each other in a flow path during partitioning, and the third bead may be introduced into the inside of the cell. Suitable examples include, for example, an embodiment in which the third bead is attached on the cell, an embodiment in which, by conjugating a subcompartment encompassing the third bead but no cell to the cell, the subcompartment is attached to the cell, an embodiment in which the third bead is ingested by the cell, or the like. Here, the positional relationship between the cell and the third bead before preparation of the second compartment includes the positional relationship between the cell and the third bead before the cell and the bead having a third barcode nucleic acid are enclosed in the second compartment, for example, in a flow path during partitioning into the second compartment.

### Step of culturing cell in coexistence with test substance

According to need, the cell or derivative thereof may be cultured in a state where it coexists with the test substance in the third compartments. The culture includes, for example, retaining the third compartments for a desired culture time and at a desired culture temperature. In the retention of the third compartments, the third compartments may be moved to and retained in a reservoir capable of retaining the plurality of types of third compartments. The above step can be performed by a known technique. For example, the step can be performed by the method described in J. J. Agresti et al., Proc Natl Acad Sci U S A., 107(9), 4004-9 (2010); A. Abbaspourrad et al., Sci Rep., 5, 12756 (2015); or B. L. Wang et al., Nat Biotechnol., 32(5), 473-8 (2014).

The culture time and the culture temperature can be set to such a culture time and a culture temperature as to enable evaluation of the response of the cell to the test substance. The culture time is, for example, 0 hours or more and 14 days or less, preferably 2 hours or more and 2 days or less. The culture time is, for example, 0°C or higher and 100°C or lower, preferably 20°C or higher and 40°C or lower.

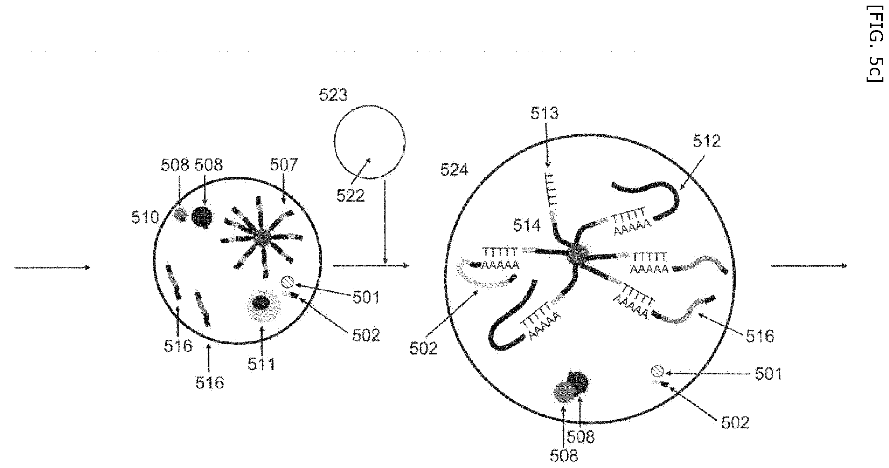

When the cell or derivative thereof is cultured in the coexistence with the test substance in the third compartments, the response of the cell to the test substance takes place during cell culture. Therefore, a step of adding a cell lysis buffer is preferably included after the culture. The above step can be performed by a known technique. For example, as shown in FIG. 5c, a fourth compartment 523 containing an aqueous solvent 522 which contains a cell lysis buffer and the third compartment 510 may be fused to form a fifth compartment 524. Examples of the fusion method include the methods described in B.L. Wang et al., Nat Biotechnol. 32(5), 473-8 (2014 May); B. Demaree et al., J Vis Exp. (135), 57598 (2018); L. Xu et al., Lab Chip. 12(20), 3936-42 (2012); and L. Mazutis et al., Lab Chip. 9(20), 2902-8 (2009).

### Step of obtaining hybridized complex

A step of obtaining a hybridized complex includes a step of cleaving the third barcode nucleic acid from the associated third bead, and hybridizing each of the genome-related nucleic acid, the first barcode nucleic acid and the third barcode nucleic acid with the second barcode nucleic acid to obtain a hybridized complex.

The above step can be performed by a known technique. For example, the step can be performed by the method described in E. Z. Macosko et al., Highly Parallel Genome-wide Expression Profiling of Individual Cells Using Nanoliter Droplets. Cell. 161, 1202-1214 (2015) or Zheng GX et al., Massively parallel digital transcriptional profiling of single cells. Nat Commun. 6; 8:14049 (2017).

One embodiment of the step of obtaining a hybridized complex in the second embodiment of the present invention will be described in accordance with FIG. 5c, without any particular limitation.

Subsequent to the above step of associating nondestructive measurement information of a cell with imaging information of a bead, and step of forming a plurality of types of third compartments, and, according to need, step of culturing the cell in the coexistence with a test substance, step of adding a cell lysis buffer, the cleavable linker is cleaved (for example, a UV-cleavable linker is cleaved by UV irradiation), a third barcode nucleic acid 516 is separated, and the cell is then lysed. Then, in the compartment, each of the first barcode nucleic acid 502, the cell-derived mRNA 512 and the third barcode nucleic acid 516 is hybridized with the second barcode nucleic acid 513 to obtain a hybridized complex 514. Then, the droplet is destroyed.

### Step of producing amplified product derived from hybridized complex

A step of producing an amplified product derived from the hybridized complex includes a step of producing an amplified product derived from the hybridized complex obtained in the above step of obtaining a hybridized complex.

The above step can be performed by a known technique. For example, the step can be performed by the method described in E. Z. Macosko et al., Highly Parallel Genome-wide Expression Profiling of Individual Cells Using Nanoliter Droplets. Cell. 161, 1202-1214 (2015) or Zheng GX et al., Massively parallel digital transcriptional profiling of single cells. Nat Commun. 6; 8:14049 (2017).

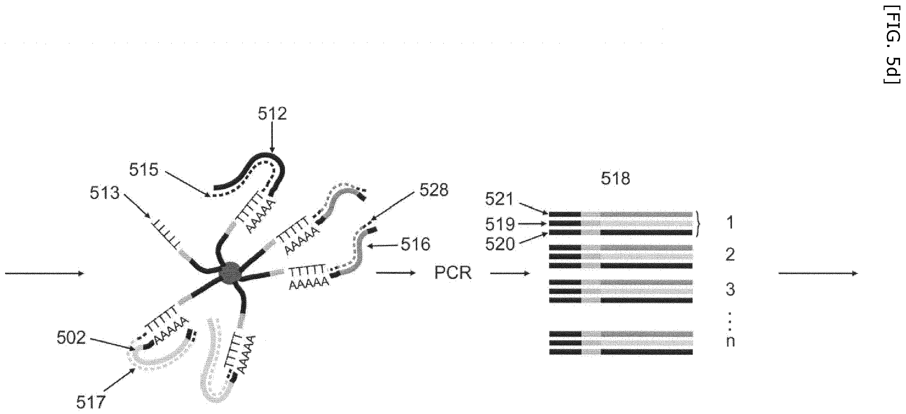

One embodiment of the step of producing an amplified product derived from the hybridized complex in the second embodiment of the present invention will be described in accordance with FIG. 5d, without any particular limitation.

Complementary strand DNA synthesis and reverse transcription reaction are performed on the hybridized complex obtained in the above step of obtaining a hybridized complex. By the synthesis and reverse transcription reactions, a cDNA 515 for the cell-derived mRNA 512 is synthesized, and a complementary strand DNA 517 for the first barcode nucleic acid 502 and a cDNA528 for the third barcode nucleic acid 516 are synthesized. Then, template switching may be performed.

Then, a PCR reaction is performed. This PCR reaction produces three types of amplified products 518 including a first amplified product 519 derived from the hybridized complex of the first barcode nucleic acid 502 with the second barcode nucleic acid 513, a second amplified product 520 derived from the hybridized complex of the cell-derived mRNA 512 with the second barcode nucleic acid 513, and a third amplified product 521 derived from the hybridized complex of the third barcode nucleic acid 516 with the second barcode nucleic acid 513. When the genome-related nucleic acid is a DNA, an extension PCR method can be performed as the above PCR reaction.

Then, based on the thus-obtained amplified product, a library of amplified products including the first, second and third amplified products derived from each of the third compartments containing the test substances 1 to n, respectively, is prepared.

### Step of integrally detecting nondestructive measurement information of cell and genome-related information in cell after coexistence with test substance

A step of integrally detecting nondestructive measurement information of a cell and genome-related information in the cell after coexistence with a test substance include a step of integrally detecting nondestructive measurement information of a cell and genome-related information in the cell after coexistence with a test substance using, as an index, an expression pattern of the amplified product obtained in the above step of producing an amplified product derived from the hybridized complex. The step further includes a step of identifying the test substance coexisting with the cell. Examples of the above expression pattern of the amplified product include sequence information of the amplified product such as sequence information of the first barcode nucleic acid (e.g., sequence information of the first common barcode region), sequence information of the second barcode nucleic acid (e.g., sequence information of the second common barcode region and sequence information of the second unique barcode region), and sequence information of the third barcode nucleic acid (e.g., sequence information of the third common barcode region and information of the third unique barcode region) in the sequence information, which are obtained by sequencing.

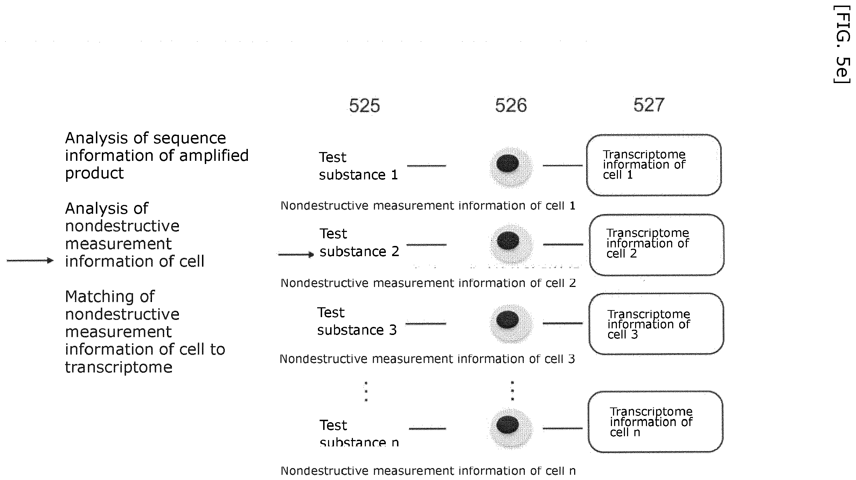

One embodiment of the step of integrally detecting nondestructive measurement information of a cell and genome-related information in the cell after coexistence with a test substance will be described in accordance with FIG. 5e, without particular limitation.

The sequence of the amplified products (the first amplified product, the second amplified product and the third amplified product) obtained in the above step of producing an amplified product derived from the hybridized complex is determined by a sequencer, and sequence information of the amplified products is analyzed. In the analysis of the second amplified product, a cell from which each amplified product is derived is assigned using sequence information of the second common barcode region as an index. Since each mRNA molecule can be discriminated by sequence information of the second unique barcode region, information such as the sequence of an mRNA for each cell and the expression amount thereof can be obtained using the sequence information as an index. Based on the information obtained by the above analysis of the second amplified product, transcriptome information 527 for each cell can be obtained.

Next, the test substance 525 coexisting with the above cell is identified. The first barcode nucleic acid corresponds to the test substance 525, as described above. Here, in the identification, the test substance 525 coexisting with the cell can be assigned to each first amplified product based on sequence information of the first common barcode region of the first barcode nucleic acid.

Next, nondestructive measurement information 526 of a cell is analyzed. Here, as mentioned above, nondestructive measurement information of the cell is associated with imaging information of the third bead, and the third barcode nucleic acid corresponding to each imaging information is linked to the third bead. Therefore, in the above analysis, based on sequence information of the third common barcode region of the third barcode nucleic acid, nondestructive measurement information 526 of a cell from which each third amplified product is derived can be assigned to each third amplified product.

Next, the test substance 525 coexisting with the cell, the nondestructive measurement information 526 of the cell and the transcriptome information 527 are matched. Therefore, one-to-one linking is possible between nondestructive measurement information of the cell coexisting with the test substance in each third compartment and genome-related information in the cell after coexistence with the test substance.

Therefore, by the above detection, the response of the cell to the coexisting test substance can be evaluated not only at the gene level, but also from the cell morphology and the like.

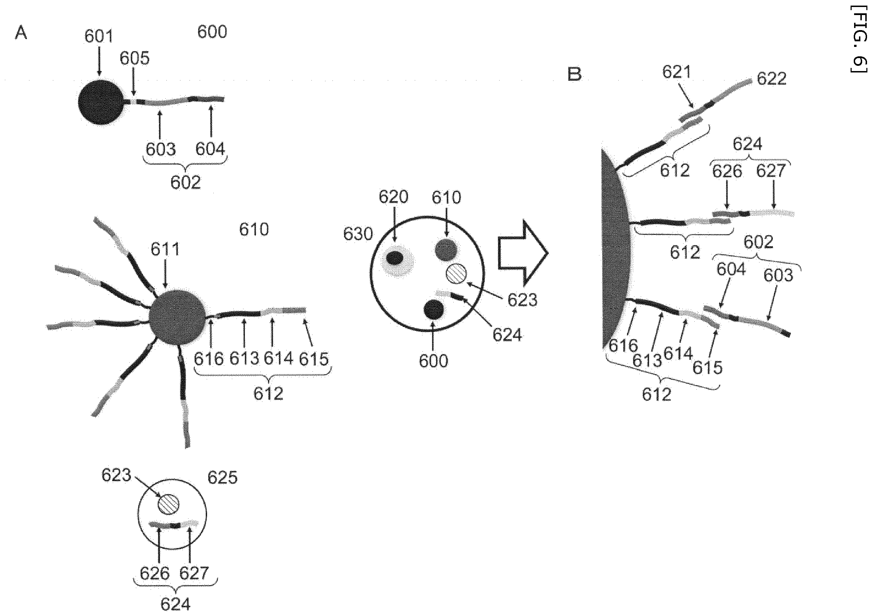

Further, FIG. 6 shows another embodiment of the first barcode nucleic acid, the second barcode nucleic acid-linked bead and the bead having a third barcode nucleic acid. FIG. 6 shows, as another embodiment, an embodiment in which each of the first barcode nucleic acid, the second barcode nucleic acid and the third barcode nucleic acid is a DNA.

In a third bead 600 having a third barcode nucleic acid in FIG. 6A, a third barcode nucleic acid 602 is linked to a third bead 601 having imaging information that can be clearly distinguished from each other. The third barcode nucleic acid 602 contains a third common barcode region 603 and a third hybridize region 604. The third barcode nucleic acid 602 contains the third common barcode region 603 and the third hybridize region 604 in this order from the third bead side. Furthermore, the third barcode nucleic acid 602 is cleavably linked to the third bead 601 via a cleavable linker 605. Furthermore, the third hybridize region 604 is a sequence complementary to a second hybridize region 615.

In a second barcode nucleic acid-linked bead 610 in FIG. 6A, a second barcode nucleic acid 612 is linked to a second bead 611. The second barcode nucleic acid 612 contains a second common barcode region 613, a second unique barcode region 614, and the second hybridize region 615. The second barcode nucleic acid 612 contains a PCR primer region 616, the second common barcode region 613, the second unique barcode region 614, and the second hybridize region 615 in this order from the second bead side. The above second hybridize region 615 is a sequence which is hybridized with a particular sequence of a genome-related nucleic acid 622 and is complementary to the sequence.

A first compartment 625 in FIG. 6A contains a test substance 1 623 and a first barcode nucleic acid 624 corresponding to the test substance 1. The first barcode nucleic acid 624 includes a first common barcode region 627 and a first hybridize region 626.

Furthermore, FIG. 6A shows a third compartment 630 containing the third bead 600 linked to a third barcode nucleic acid, the second barcode nucleic acid-linked bead 610, the test substance 1 623, the first barcode nucleic acid 624, and a cell 620.

FIG. 6B shows a hybridized complex. Here, the second barcode nucleic acid 612 is hybridized with the third hybridize region 604 of the third barcode nucleic acid 602 in the second hybridize region 615. Furthermore, the second barcode nucleic acid 612 is hybridized with a particular sequence 621 of the genome-related nucleic acid 622 in the second hybridize region 615. Further, the second barcode nucleic acid 612 is hybridized with the first hybridize region 626 of the first barcode nucleic acid 624 in the second hybridize region 615.

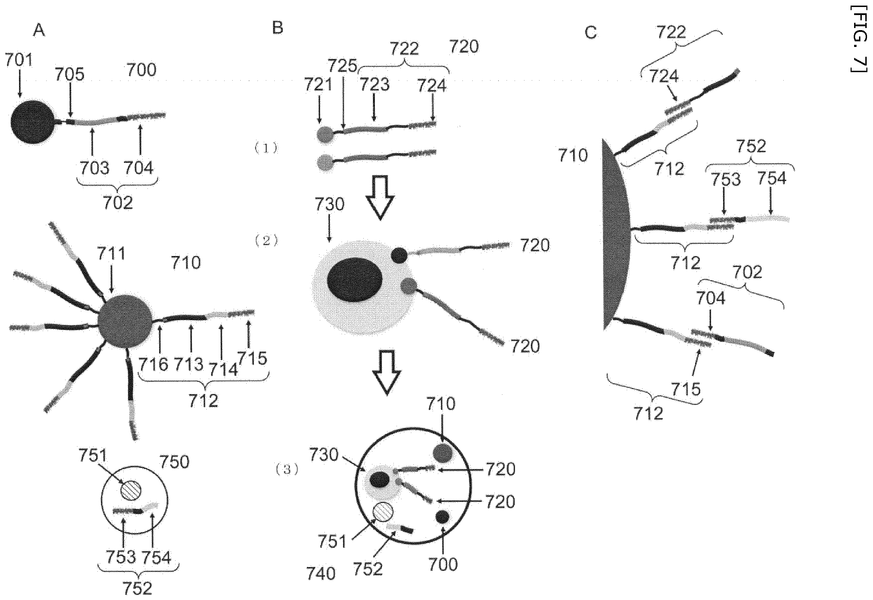

Further, FIG. 7 shows another embodiment of the first barcode nucleic acid, the bead having a third barcode nucleic acid, and the second barcode nucleic acid-linked bead. FIG. 7 shows an embodiment in which each of the first barcode nucleic acid and the second barcode nucleic acid is a DNA and a nucleic acid probe 720 which is specific to a molecule such as a protein expressed in a cell (hereinafter also referred to as nucleic acid probe 720) is included.

In a third bead 700 having a third barcode nucleic acid in FIG. 7A, a third barcode nucleic acid 702 is linked to a third bead 701 having imaging information that can be clearly distinguished from each other. The third barcode nucleic acid 702 contains a third common barcode region 703 and a third hybridize region 704. The third barcode nucleic acid 702 contains the third common barcode region 703 and the third hybridize region 704 in this order from the third bead side. Furthermore, the third barcode nucleic acid 702 is cleavably linked to the third bead 701 via a cleavable linker 705. Furthermore, the third hybridize region 704 is a sequence complementary to the second hybridize region 715.

In a second barcode nucleic acid-linked bead 710 in FIG. 7A, a second barcode nucleic acid 712 is linked to a second bead 711. The second barcode nucleic acid 712 contains a second common barcode region 713, a second unique barcode region 714, and the second hybridize region 715. The second barcode nucleic acid 712 contains a PCR primer region 716, the second common barcode region 713, the second unique barcode region 714, and the second hybridize region 715 in this order from the second bead side. The above second hybridize region 715 is a sequence complementary to a hybridize region 724 of a nucleic acid probe 720.

A first compartment 750 in FIG. 7A contains a test substance 1 751 and a first barcode nucleic acid 751 corresponding to the test substance 1. The first barcode nucleic acid 752 includes a first common barcode region 754 and a first hybridize region 753.

FIG. 7B(1) shows the nucleic acid probe 720. FIG. 7B(2) shows an embodiment in which the above nucleic acid probe 720 is bound to a protein expressed in a cell 730 obtained by mixing the above nucleic acid probe 720 with the cell 730 and washing. Furthermore, FIG. 7B(3) shows a third compartment 740 containing the cell 730 to which the above nucleic acid probe 720 is bound, together with the third bead 700 having a third barcode nucleic acid, the second barcode nucleic acid-linked bead 710, the test substance 1 751, and the first barcode nucleic acid 752.

In the nucleic acid probe 720 in FIG. 7B(1), a fourth barcode nucleic acid 722 is linked to a molecule 721 which binds specifically to a molecule such as a target protein expressed in a cell (hereinafter also referred to as binding molecule 721). The fourth barcode nucleic acid 722 contains a fourth common barcode region 723 and a fourth hybridize region 724. The fourth barcode nucleic acid 722 contains the fourth common barcode region 723 and the fourth hybridize region 724 in this order from the binding molecule 721. Furthermore, the fourth barcode nucleic acid 722 is cleavably linked to the binding molecule 721 via a cleavable linker 725. Furthermore, the fourth hybridize region 724 is a sequence complementary to the second hybridize region 715. The above nucleic acid probe 720 may be plural types of probes containing the fourth barcode nucleic acid 722 different for each binding molecule 721.

FIG. 7C shows a hybridized complex. The second barcode nucleic acid 712 is hybridized with the third hybridize region 704 of the third barcode nucleic acid 702 in the second hybridize region 715. Further, the second barcode nucleic acid 712 is hybridized with the fourth hybridize region 724 of the fourth barcode nucleic acid 722 in the second hybridize region 715. Further, the second barcode nucleic acid 712 is hybridized with the first hybridize region 753 of the first barcode nucleic acid 752 in the second hybridize region 715. Analysis of sequence information of an amplified product derived from the sequence of the fourth common barcode region 723 of the above nucleic acid probe 720 enables confirmation of the presence or absence of expression or the amount of expression of a target molecule in the cell after coexistence with the test substance.

Therefore, use of a nucleic acid probe specific to a molecule expressed in a cell enables one-to-one correspondence between a large amount of proteomics in the cell after coexistence with the test substance and nondestructive measurement information of the cell.

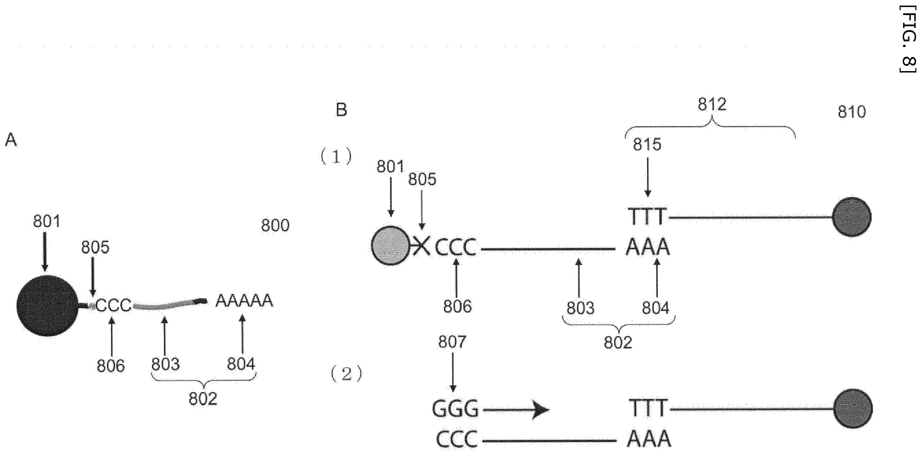

Furthermore, another embodiment of the bead having the third barcode nucleic acid is shown in FIG. 8. FIG. 8 shows, as another embodiment, an embodiment in which the third barcode nucleic acid is a DNA and contains a particular sequence.

In a bead 800 linked to a third barcode nucleic acid in FIG. 8A, a third barcode nucleic acid 802 is linked to a third bead 801 having imaging information that can be clearly distinguished from each other. The third barcode nucleic acid 802 contains a third common barcode region 803 and a third hybridize region 804. The third barcode nucleic acid 802 contains the third common barcode region 803 and the third hybridize region 804 in this order from the third bead side. Furthermore, the third barcode nucleic acid 802 is cleavably linked to the third bead 801 via a cleavable linker 805. Furthermore, a particular sequence 806 is contained between the third barcode nucleic acid 803 and the cleavable linker 805. Furthermore, the third hybridize region 804 is a sequence complementary to the second hybridize region 815.

Further, FIG. 8B(1) shows an embodiment of hybridization between the third barcode nucleic acid and the second barcode nucleic acid in the hybridization complex. 810 represents a second barcode nucleic acid linked-bead. Here, the second barcode nucleic acid 812 is hybridized with the third hybridize region 804 of the third barcode nucleic acid 802 in the second hybridize region 815. Furthermore, as shown in FIG. 8B(2), by using a primer 807 having a sequence complementary to a particular sequence 806, DNAs complementary to the third barcode nucleic acid 802 and the second barcode nucleic acid 812 are synthesized, and further, a DNA complementary to the second barcode nucleic acid 812 is synthesized by template switching.

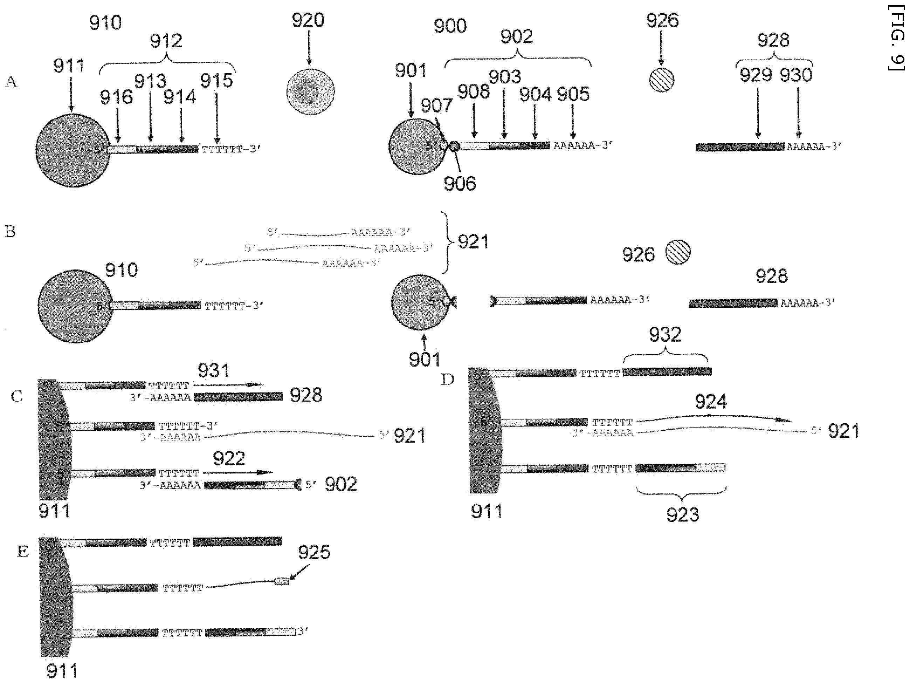

Further, FIG. 9 shows another embodiment of the first barcode nucleic acid, the second barcode nucleic acid-linked bead and the bead having a third barcode nucleic acid. FIG. 9 shows, as another embodiment, an embodiment in which each of the first barcode nucleic acid, the second barcode nucleic acid and the third barcode nucleic acid is a DNA.

FIG. 9A shows the step of preparing a compartment. In a third bead 900 linked to a third barcode nucleic acid in FIG. 9A, a third barcode nucleic acid 902 is linked to a third bead 901 having imaging information that can be clearly distinguished from each other. The third barcode nucleic acid 902 contains a third common barcode region 903 and a third hybridize region 905. The third barcode nucleic acid 902 contains a PCR primer region 908, the third common barcode region 903, the third unique barcode region 904, and the third hybridize region 905 in this order from the third bead side. Furthermore, the third barcode nucleic acid 902 is cleavably linked via a cleavable linker 906 to an acrylamide moiety 907 such as an acrylic phosphoramidite moiety (Acrydite (trademark)) bound to the third bead 901. Furthermore, the third hybridize region 905 is polyadenine.

In a second barcode nucleic acid-linked bead 910 in FIG. 9A, a second barcode nucleic acid 912 is linked to a second bead 911. The second barcode nucleic acid 912 contains a second common barcode region 913, a second unique barcode region 914, and a second hybridize region 915. The second barcode nucleic acid 912 contains a PCR primer region 916, the second common barcode region 913, the second unique barcode region 914, and the second hybridize region 915 in this order from the second bead side. The above second hybridize region 915 is polythymine. 920 represents a cell.

Also, a first barcode nucleic acid 928 in FIG. 9A includes a first common barcode region 929 and a first hybridize region 930. Here, the first hybridize region 930 is polyadenine. 926 represents the test substance 1.

FIGs. 9B to 9E show the step of obtaining a hybridized complex after the step of associating nondestructive measurement information of a cell with imaging information of a bead.

In FIG. 9B, a photocleavable linker is cleaved, and a cell is lysed. FIG. 9B shows a third bead 901 in which a third barcode nucleic acid 902 is cleaved, a second barcode nucleic acid-linked bead 910, and a cell-derived mRNA 921.

FIG. 9C shows a hybridized complex. Here, the second barcode nucleic acid 912 is hybridized with a first hybridize region 930 of a first barcode nucleic acid 928 in a second hybridize region 915. Furthermore, a complementary strand DNA 932 is synthesized by a DNA polymerase 931. Here, the second barcode nucleic acid 912 is hybridized with the third hybridize region 905 of the third barcode nucleic acid 902 in the second hybridize region 915. Furthermore, a complementary strand DNA 923 is synthesized by a DNA polymerase 922. Also, the second barcode nucleic acid 912 is hybridized with polyadenine of the cell-derived mRNA 921 in the second hybridize region 915.

In FIG. 9D, in the hybridized complex, a reverse transcription is performed, and a cDNA 924 for the cell-derived mRNA 921 is synthesized.

In FIG. 9E, the 3' end of the synthesized cDNA is tagged with a DNA tag 925. The above DNA tag can be used as a PCR primer site, and examples thereof include a transposon Mosaic End (ME) sequence.

### Partitioning

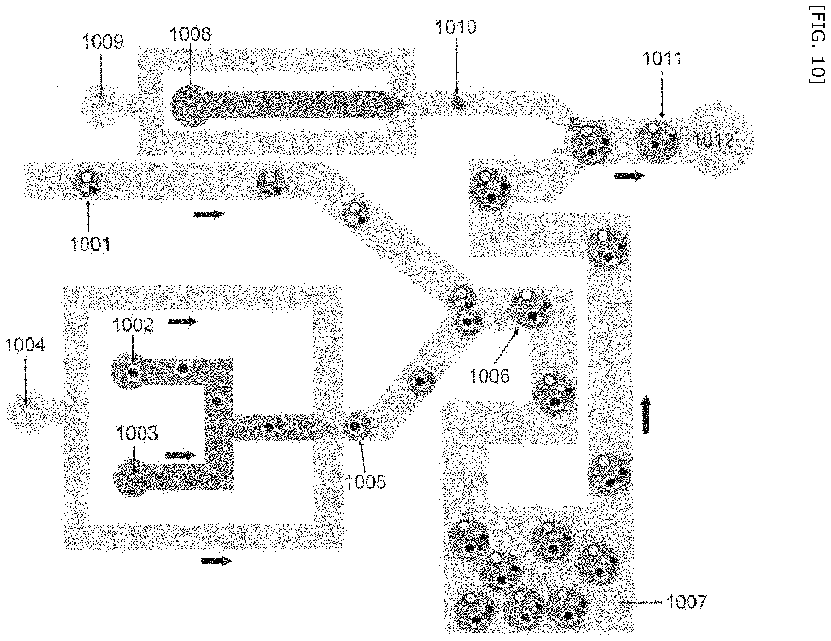

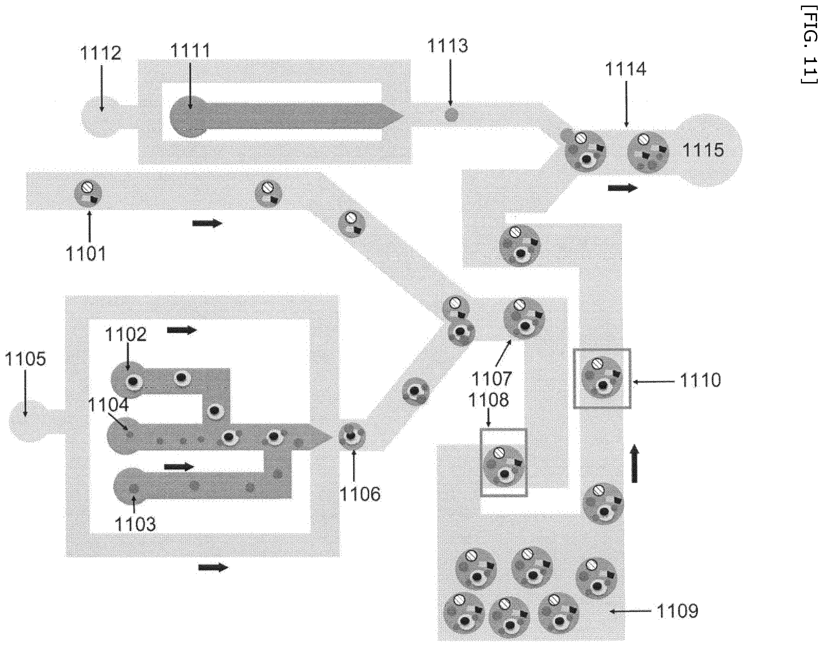

Partitioning in the present invention, namely, one embodiment of a method for producing a compartment is specifically described based on FIGs. 10 and 11.

FIG. 10 shows a method for producing a compartment in an embodiment including a test substance, a first barcode nucleic acid, a cell and a second barcode nucleic acid-linked bead.

Specifically, a plurality of types of first compartments 1001 containing test substances 1 to n and first barcode nucleic acids 1 to n corresponding to the test substances 1 to n are prepared first. On the other hand, a solution containing a cell 1002 and a second barcode nucleic acid-linked bead 1003 is put into an oil 1004 to form a droplet as a second compartment 1005. Then, a first compartment 1001 and a second compartment 1005 are fused to form a third compartment 1006. The obtained third compartment 1006 is moved to a reservoir 1007 and cultured for a certain period of time. On the other hand, an aqueous solvent 1008 containing a cell lysis buffer is released into a flow path filled with oil 1009 to form a fourth compartment 1010. Then, the fourth compartment 1010 and the third compartment 1006 are fused to form a fifth compartment 1011, which is released at an outlet 1012. Examples of the apparatus specifically used to produce a compartment in the above method include an apparatus used for a droplet production method using microfluidic techniques such as a flow focusing device. As the above apparatus, a known apparatus can be used as long as the present invention is not impaired. Examples of the apparatus used for a droplet production method using microfluidic techniques such as a flow focusing device include the apparatuses mentioned in E. Z. Macosko et al., Highly Parallel Genome-wide Expression Profiling of Individual Cells Using Nanoliter Droplets. Cell. 161, 1202-1214 (2015), Microfluid Nanofluid (2008) 5:585-594, Applied Physics Letters, Vol.85, No.13, 27, September 2004, p2649-2651, T. M. Gierahn et al., Seq-Well: portable, low-cost RNA sequencing of single cells at high throughput, Nature Methods, 14, 395-398 (2017), and JP 2013-508156 W and the like.

FIG. 11 shows a method of producing a compartment and detecting and/or measuring both nondestructive measurement information of a cell and imaging information of a third bead in an embodiment including a test substance, a first barcode nucleic acid, a cell, a second barcode nucleic acid-linked bead, and a bead having a third barcode nucleic acid.

Specifically, a plurality of types of first compartments 1101 containing test substances 1 to n and first barcode nucleic acids 1 to n corresponding to the test substances 1 to n are prepared first. On the other hand, a solution containing a cell 1102, a second barcode nucleic acid-linked bead 1103, and a bead 1104 having a third barcode nucleic acid is put into an oil 1105 to form a droplet as a second compartment 1106. Then, a first compartment 1101 and a second compartment 1106 are fused to form a third compartment 1107. The obtained third compartment 1107 is moved to a reservoir 1109 and cultured for a certain period of time. On the other hand, an aqueous solvent 1111 containing a cell lysis buffer is released into a flow path filled with oil 1112 to form a fourth compartment 1113. Then, the fourth compartment 1113 and the third compartment 1107 are fused to form a fifth compartment 1114, which is released at an outlet 1115. Nondestructive measurement information is measured before and after culture in the third compartment. It is possible to perform one type of measurement each before and after culture, for example, fluorescence imaging measurement 1108 and bright field imaging 1110. Here, in the fluorescence imaging measurement before culture, accurate imaging information of a cell and a third bead can be obtained, and, in the bright field imaging after culture, a combination of the cell after coexistence with the test substance and the third bead can be confirmed. Further, according to the method, it becomes possible to photograph a natural state of the cell and the third bead. Further, fluorescence imaging measurement and bright field imaging measurement may be performed before culture, and unmodified imaging measurement such as Raman may be used. In the above methods, examples of an apparatus specifically used include an apparatus used for a droplet production method using microfluidic techniques such as a flow focusing device as described above for production of a compartment, and imaging flow cytometry for measurement of nondestructive measurement information. As the above apparatus, a known apparatus can be used as long as the present invention is not impaired. According to the above apparatus used for a droplet production method using microfluidic techniques such as a flow focusing device, it is possible to efficiently produce a large amount of beads having imaging information including color, shape, and size.

### Other embodiments

In the method of the present invention, the third compartment may be produced by fusing the first and second compartments as described above, but the combinations of the components to be enclosed in the first and second compartments and the method for producing the third compartment can be appropriately changed and implemented, as long as the association between the test substance and the first barcode nucleic acid and the association between the second barcode nucleic acid and the cell or genome derivative thereof are not impaired. Thus, according to another preferred embodiment of the present invention, there is provided a method for detecting genome-related information of a cell or a derivative thereof coexisting with at least one type of test substance, including:
preparing a plurality of target compartments containing a cell or a derivative thereof coexisting with the test substance, a first barcode nucleic acid, and a second barcode nucleic acid-linked bead,
wherein the first barcode nucleic acid is preliminarily associated with the test substance, and
the second barcode nucleic acid-linked bead contains a plurality of second barcode nucleic acids hybridizable with a genome-related nucleic acid corresponding to a cell genome or a derivative thereof or the first barcode nucleic acid;
hybridizing each of the genome-related nucleic acid and the first barcode nucleic acid with the second barcode nucleic acid to obtain a hybridized complex;
producing an amplified product derived from the hybridized complex; and
detecting genome-related information in the cell after coexistence with the test substance using an expression pattern of the amplified product as an index. In another preferred embodiment, the target compartment corresponds to the first compartment in the first embodiment.

In another preferred embodiment, the association of the first barcode nucleic acid with the test substance preferably includes making the test substance coexist with the cell or derivative thereof and the first barcode nucleic acid. When the cell or derivative thereof is made to coexist with the first barcode nucleic acid, the surface of the cell or derivative thereof is preferably bound to the first barcode nucleic acid by a linker or the like, from the viewpoint of stable production of the target compartments.

The preparation of the target compartments preferably includes binding a subcompartment that contains the cell or derivative thereof made to coexist with the test substance and the first barcode nucleic acid, to a subcompartment that contains the second barcode nucleic acid-linked bead.

According to still another preferred embodiment, a bead having a third barcode nucleic acid can be used to integrally detect nondestructive measurement information and genome-related information of a cell coexisting with at least one type of test substance. According to still another preferred embodiment, a method for integrally detecting nondestructive measurement information and genome-related information of a cell coexisting with at least one type of test substance,
the target compartment further containing a bead having a third barcode nucleic acid,
wherein each bead having a third barcode nucleic acid is:
   a particle cleavably linked to a third barcode nucleic acid corresponding to each imaging information or
   an organism containing a third barcode nucleic acid corresponding to each imaging information, and
imaging information of the bead having a third barcode nucleic acid can be clearly distinguished from each other,
the method further including:
   detecting both nondestructive measurement information of the cell and imaging information of the bead having a third barcode nucleic acid and associating the nondestructive measurement information of the cell with the imaging information of the bead having a third barcode nucleic acid;
   cleaving or taking out the third barcode nucleic acid from the associated bead having the third barcode nucleic acid, and hybridizing each of the genome-related nucleic acid and the third barcode nucleic acid with the second barcode nucleic acid to obtain a hybridized complex;
   producing an amplified product derived from the hybridized complex; and
   integrally detecting nondestructive measurement information and genome-related information of the cell using an expression pattern of the amplified product as an index.

Further details of the above other preferred embodiments can be carried out according to the first embodiment and the second embodiment.

### Combination

Another embodiment of the present invention is a combination of a first barcode nucleic acid and a second barcode nucleic acid-linked bead for detecting genome-related information of a cell coexisting with at least one type of test substance, wherein the embodiment is characterized that:
each first barcode nucleic acid corresponds to the at least one type of test substance coexisting with the cell;
the second barcode nucleic acid-linked bead is linked to a plurality of second barcode nucleic acids hybridizable with a genome-related nucleic acid corresponding to a cell genome or a derivative thereof or the first barcode nucleic acid; and
the test substance can be identified using an expression pattern of a first amplified product derived from a hybridized complex of the first barcode nucleic acid with the second barcode nucleic acid as an index, and the genome-related information of the cell can be detected using an expression pattern of a second amplified product derived from a hybridized complex of the genome-related nucleic acid with the second barcode nucleic acid as an index. The combination of the present invention is not particularly limited as long as it is used for detecting genome-related information of a cell coexisting with at least one type of test substance, and the combination may be in a form of one composition or an agent such as a reagent, or may be composed of a combination of a plurality of compositions or agents such as reagents. The above combination may be composed integrally or as separate body. Examples of the above combination of a plurality of compositions or compositions as separate body include a combination of a composition including the first barcode nucleic acid and a composition including the second barcode nucleic acid-linked bead. Here, the above combination may be a reagent kit to be used for a method for detecting genome-related information of a cell coexisting with test substance of at least one type of cell. The reagent kit may be provided with a buffer, a reagent necessary for reverse transcription or PCR reaction, a necessary reagent such as a cell lysis buffer, instructions for use or the like.

### Detecting agent including first barcode nucleic acid

Another embodiment of the present invention is a detecting agent containing a first barcode nucleic acid, which is used together with a second barcode nucleic acid-linked bead, for detecting genome-related information of a cell coexisting with at least one type of test substance, wherein the embodiment is characterized that:
each first barcode nucleic acid corresponds to the at least one type of test substance coexisting with the cell;
the second barcode nucleic acid-linked bead is linked to a plurality of second barcode nucleic acids hybridizable with a genome-related nucleic acid corresponding to a cell genome or a derivative thereof or the first barcode nucleic acid; and
the test substance can be identified using an expression pattern of a first amplified product derived from a hybridized complex of the first barcode nucleic acid with the second barcode nucleic acid as an index, and the genome-related information of the cell can be detected using an expression pattern of a second amplified product derived from a hybridized complex of the genome-related nucleic acid with the second barcode nucleic acid as an index.

### Detecting agent including second bead

Another embodiment of the present invention is a detecting agent containing a second barcode nucleic acid-linked bead, which is used together with a first barcode nucleic acid, for detecting genome-related information of a cell coexisting with at least one type of test substance, wherein the embodiment is characterized that:
each first barcode nucleic acid corresponds to the at least one type of test substance coexisting with the cell;
the second barcode nucleic acid-linked bead is linked to a plurality of second barcode nucleic acids hybridizable with a genome-related nucleic acid corresponding to a cell genome or a derivative thereof or the first barcode nucleic acid; and
the test substance can be identified using an expression pattern of a first amplified product derived from a hybridized complex of the first barcode nucleic acid with the second barcode nucleic acid as an index, and the genome-related information of the cell can be detected using an expression pattern of a second amplified product derived from a hybridized complex of the genome-related nucleic acid with the second barcode nucleic acid as an index.

### Composition including a plurality of types of first compartments

Another embodiment of the present invention is a composition including a plurality of types of first compartments, wherein the embodiment is characterized that each first compartment contains one type of test substance and a first barcode nucleic acid corresponding to the one type of test substance. The composition can be used as a composition for use in screening, that is, as a library. According to another preferred embodiment of the present invention, the above composition including a plurality of types of first compartments may be a composition including first compartments, which are used together with a second barcode nucleic acid-linked bead, for detecting genome-related information of a cell coexisting with at least one type of test substance. Each first barcode nucleic acid corresponds to the at least one type of test substance coexisting with the cell. The second barcode nucleic acid-linked bead is linked to a plurality of second barcode nucleic acids hybridizable with a genome-related nucleic acid corresponding to a cell genome or a derivative thereof or the first barcode nucleic acid. The test substance can be identified using an expression pattern of a first amplified product derived from a hybridized complex of the first barcode nucleic acid with the second barcode nucleic acid as an index, and the genome-related information of the cell can be detected using an expression pattern of a second amplified product derived from a hybridized complex of the genome-related nucleic acid with the second barcode nucleic acid as an index. The form of the first compartments in the composition containing the plurality of types of first compartments is not particularly limited, and, when the form of the first compartments is an aqueous droplet in oil, the above composition may contain oil together with an aqueous droplet.

### Composition including a plurality of types of second compartments

Another embodiment of the present invention is a composition including a plurality of types of second compartments, which are used together with a first barcode nucleic acid, for detecting genome-related information of a cell coexisting with at least one type of test substance, wherein the embodiment is characterized that:
each first barcode nucleic acid corresponds to the at least one type of test substance coexisting with the cell;
each second compartment contains a cell or a derivative thereof and a second barcode nucleic acid-linked bead;
the second barcode nucleic acid-linked bead is linked to a plurality of second barcode nucleic acids hybridizable with a genome-related nucleic acid corresponding to a cell genome or a derivative thereof or the first barcode nucleic acid; and
the test substance can be identified using an expression pattern of a first amplified product derived from a hybridized complex of the first barcode nucleic acid with the second barcode nucleic acid as an index, and the genome-related information of the cell can be detected using an expression pattern of a second amplified product derived from a hybridized complex of the genome-related nucleic acid with the second barcode nucleic acid as an index. Still another embodiment of the present invention is a composition including a plurality of types of second compartments, which are used together with a first barcode nucleic acid, for integrally detecting nondestructive measurement information and genome-related information of a cell coexisting with at least one type of test substance, wherein the embodiment is characterized that:
   each first barcode nucleic acid corresponds to the at least one type of test substance coexisting with the cell;
   each second compartment contains a cell or a derivative thereof, a second barcode nucleic acid-linked bead and a bead having a third barcode nucleic acid;
   the second barcode nucleic acid-linked bead is linked to a plurality of second barcode nucleic acids hybridizable with a genome-related nucleic acid corresponding to a cell genome or a derivative thereof, the first barcode nucleic acid or the third barcode nucleic acid; and
   each bead having a third barcode nucleic acid is:
      a particle cleavably linked to a third barcode nucleic acid corresponding to each imaging information or
      an organism containing a third barcode nucleic acid corresponding to each imaging information, and
   imaging information of the bead having a third barcode nucleic acid in a third compartment can be clearly distinguished from each other, and
   the test substance can be identified using an expression pattern of a first amplified product derived from a hybridized complex of the first barcode nucleic acid with the second barcode nucleic acid as an index, and the genome-related information of the cell can be detected using an expression pattern of a second amplified product derived from a hybridized complex of the genome-related nucleic acid with the second barcode nucleic acid as an index;
   both nondestructive measurement information of the cell and imaging information of the bead having a third barcode nucleic acid are detected;
   the nondestructive measurement information of the cell is associated with the imaging information of the bead having a third barcode nucleic acid;
   nondestructive measurement information of the cell can be detected using an expression pattern of a third amplified product derived from a hybridized complex of the third barcode nucleic acid with the second barcode nucleic acid as an index; and
   nondestructive measurement information and genome-related information of a cell coexisting with the test substance can be integrally detected. The above composition including the plurality of types of second compartments can be used as a composition for screening for a test substance. The form of the second compartments in the composition containing the plurality of types of second compartments is not particularly limited, and, when the form of the second compartments is an aqueous droplet in oil, the above composition may contain oil together with an aqueous droplet.

### Method for screening for test substance

According to a preferred embodiment of the present invention, there is provided a method for screening for a test substance, based on the genome-related information of the cell or derivative thereof detected by the detection method of the first embodiment of the present invention. According to a preferred embodiment of the present invention, there is provided a method for screening for a test substance, based on the nondestructive measurement information and the genome-related information of the cell detected by the detection method of the second embodiment of the present invention.

Any of the above embodiments of the combination, the detecting agent including a first bead, the detecting agent including a second bead, composition and screening method can be performed according to the description on the detection method of the present invention.

### EXAMPLES

The present invention will be specifically described by way of Examples, but the present invention is not limited to these Examples. Unless otherwise specified, the measurement methods of the present invention and the units are in accordance with the provisions of the Japanese Industrial Standards (JIS).

### Example 1: Production test of compartment

In this experiment, a microfluidic device was used to confirm that a droplet simultaneously containing a drug (Lipoporysaccharide: LPS), a cell (THP1 cell) and a second barcode nucleic acid-linked bead (Macosko-2011-10 (V+), manufactured by ChemGenes (trademark)) was generated and could exist stably. For generation of a droplet (compartment) in the present Example, a flow focusing device was used according to the description of E. Z. Macosko et al., Highly Parallel Genome-wide Expression Profiling of Individual Cells Using Nanoliter Droplets. Cell. 161, 1202-1214 (2015). An RPMI1640 medium supplemented with 10% FBS was used as the aqueous phase, and LPS was dissolved at a concentration of 2 µg/mL. Droplet Generator oil for EvaGreen (manufactured by BioRad) was used in the organic solvent phase. The Droplet Generator oil for EvaGreen used in the organic solvent phase has oxygen permeability and is suitable for in-droplet culture of cells.

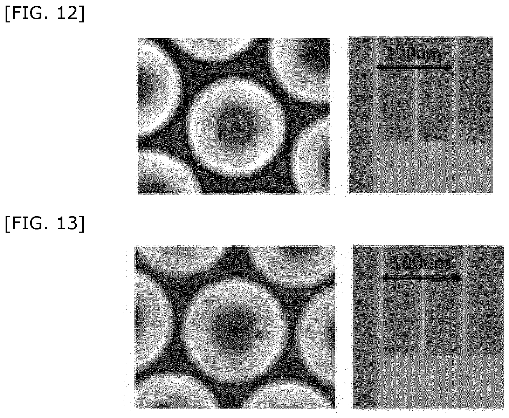

In the experiment, when the above components were mixed in an aqueous phase and then poured into the microfluidic device, it could be confirmed, from a micrograph, that fine and uniform droplets (compartments) enclosing a plurality of components were generated and could exist stably, as shown in FIG. 12. In FIG. 12, the substance present in the center of the photograph is the second barcode nucleic acid-linked bead, and the substances floating around it are the cells.

When a bead having a third barcode nucleic acid, in addition to the above components, was mixed in an aqueous phase and then poured into the microfluidic device, it could be confirmed, from a micrograph, that fine and uniform droplets (compartments) enclosing a plurality of components were generated and could exist stably, as shown in FIG. 13. In FIG. 13, the substance present in the center of the photograph is the second barcode nucleic acid-linked bead, the substances floating around the second barcode nucleic acid-linked bead are the cells, and a light-colored spherical body observed at the peripheral edge part of the droplet is the bead having a third barcode nucleic acid.

From the above experiment, it was confirmed that a compartment in which the cell, the second barcode nucleic acid-linked bead, and the bead having a third barcode nucleic acid were enclosed could be produced stably.

Further, since it was possible to enclose a combination of different components in a plurality of subcompartments to produce a compartment in which a cell, a second barcode nucleic acid-linked bead, and a bead having a third barcode nucleic acid were encapsulated, and the droplets were stable even when such a plurality of components were contained, it was confirmed whether genome-related information of the cell coexisting with the drug (LPS) could be detected by further using a first barcode nucleic acid corresponding to the drug (LPS).

### Example 2: Confirmation test of detection of genome-related information of cell coexisting with drug (LPS)

In this experiment, first, in a tube 1, an oligonucleotide linker added with a cholesterol modification (hereinafter, the oligonucleotide linker added with a cholesterol modification is also referred to as "anchor CMO") (5'-3' Cholesterol-TEG-GTAACGATGGAGCTGTCACTTGGAATTCTCGGGTGCCAAGG)-3' (SEQ ID NO: 1)) and a cell were mixed in water. A commercially available product mentioned in http://sg.idtdna.com/site/Catalog/Modifications/Product/2555 is used as the "3' Cholesterol-TEG" in the oligonucleotide linker. Further, a THP1 cell was used as the cell, the cell concentration was 1×10⁷ /mL, Phosphate Buffer Saline (PBS) was used as a solvent, and the final concentration of the anchor CMO was set to 250 nM. Incubation was performed at 4°C for 5 minutes. A co-oligonucleotide linker added with the other cholesterol modification (anchor CMO: 5'-AGTGACAGCTGGATCGTTAC-3' Cholesterol-TEG-3' (SEQ ID NO: 2)) was further mixed with the product. The final concentration of the co-anchor CMO was set to 250 nM. Incubation was performed at 4°C for 5 minutes.

Next, an aqueous solution of an oligonucleotide containing a first barcode nucleic acid A (8 bases) was mixed in the tube 1 and incubated. In the cell used at this time, the oligonucleotide containing the first barcode nucleic acid A was 5'-CCTTGGCACCCGAGAATTCCACCACCATGA₃₀-3' (SEQ ID NO: 3). Here, A₃₀ added to the end of the first barcode nucleic acid A is polyadenine (poly (A₃₀)) consisting of 30 residues. The final concentration of the first barcode nucleic acid A was set to 250 nM. Incubation was performed at 4°C for 5 minutes.

Further, in the tube 2, a cell and an oligonucleotide containing a first barcode nucleic acid B (8 bases) were mixed in PBS by the same method and under the same conditions as in the tube 1 to obtain an aqueous solution. The oligonucleotide containing the first barcode nucleic acid B (8 bases) used was 5'-CCTTGGCACCCGAGAATTCCATGAGACCTA₃₀-3' (SEQ ID NO: 4).

The cell was then resuspended in an RPMI-1640 medium containing 10% FBS and 50 µM 2-mercaptoethanol in each tube. Only to the tube 1, Lipopolysaccharide (LPS) suspended in Dimethyl Sulfoxide (DMSO) was added as a drug at a final concentration of 2 µg/mL. Only DMSO, which is a solvent for the drug, was added to a tube 2. The tube 1 and tube 2 were subjected to incubation at 37°C under CO₂ for 1 hour. Through the experiment so far, the drug conditions of containing the drug LPS (tube 1) and containing no drug (tube 2) were made to corresponded to the first barcode nucleic acids A and B, respectively.

Next, the first barcode nucleic acid A and the cell in the tube 1 were compartmentalized to obtain a compartment A containing the first barcode nucleic acid A and the cell, and further, the first barcode nucleic acid B and the cell in the tube 2 were compartmentalized to obtain a compartment B containing the first barcode nucleic acid B. Further, the compartment A and the compartment B were mixed so as to attain a cell ratio of 95:5, thereby obtaining a compartment mixture X, and the compartment A and the compartment B were mixed so as to attain a cell ratio of 1:1, thereby obtaining a compartment mixture Y.

Eighty (80) uL of a solution containing about 4800 cells was dispensed from each of the compartment mixtures X and Y, and one cell analysis was performed. For readout of the first barcode nucleic acid corresponding to each drug, the single cell 3' reagent kit v3 manufactured by 10x Genomics was used as in the case of the Chromium controller device manufactured by 10x Genomics, which is a one cell analysis technique using droplet technology. In this technique, a large number of droplets (compartments) are formed in a microchannel, and, a second barcode nucleic acid-linked bead and one cell, which are different for each droplet, are mixed in each one droplet probabilistically at 1:1. That is, both the compartment mixture X and the compartment mixture Y were treated so that the second barcode nucleic acid-linked bead was enclosed in each compartment probabilistically at 1:1. Here, in the second barcode nucleic acid-linked bead used in the above kit, a plurality of second barcode nucleic acids are linked to the second bead via a linker. Further, each of the plurality of second barcode nucleic acids linked to the second bead includes a second common barcode region (16 bases) which is in common with each other, a second unique barcode region (12 bases) which can be clearly distinguished from each other, and a second hybridize region hybridizable with the nucleic acid derived from the cell genome or the first barcode nucleic acid.

The compartment was allowed to stand so that the first hybridize region (poly (A)) at the end of the first barcode nucleic acid corresponding to the drug and the poly (A) end of the cell-derived mRNA are each bound to the second hybridize region (poly (dT) sequence) of the second barcode nucleic acid-linked bead. Furthermore, a reverse transcription reaction using a reverse transcriptase was carried out in each droplet, and each complex was treated with a primer 5'-CTTGGCACCCGAGAATTCC-3' (SEQ ID NO: 5) for the first barcode nucleic acid and a complementary strand DNA primer contained in the single cell 3' reagent kit v3 manufactured by 10x Genomics to generate each complementary strand DNA.

The droplets were then destroyed in a state where the individual droplets were mixed, the complementary strand DNA group extracted from each droplet was amplified by PCR reaction, and the DNA concentration was measured by Qubit Fluorometer manufactured by Invitrogen. The compartment mixture X was 23.4 ng/µL, and the compartment mixture Y was 30.4 ng/µL.

Next, for each cell, a sequence library containing a first amplified product derived from a hybridized complex of the first barcode nucleic acid and the second barcode nucleic acid, and a second amplified product derived from a hybridized complex of the genome-related nucleic acid and the second barcode nucleic acid was prepared by a PCR reaction. 5'-AATGATACGGCGACCACCGAGATCTACACTCTTTCCCTACACGACGCTCTTC CGATCT-3' (SEQ ID NO: 6) was used as a primer on the Read1 side (Universal I5 primer). As a primer on the Read2 side (TruSeq RPI primer), 5'-CAAGCAGAAGACGGCATACGAGATATTGGCGTGACTGGAGTTCCTTGGCAC CCGAGAATTCCA-3' (SEQ ID NO: 7) was used for the compartment mixture X, and 5'-CAAGCAGAAGACGGCATACGAGATTACAAGGTGACTGGAGTTCCTTGGCAC CCGAGAATTCCA-3' (SEQ ID NO: 8) was used for the compartment mixture Y. For the obtained next-generation sequence library, the DNA size and concentration were measured using a D5000 screen tape manufactured by Agilent, and the quality of the library was confirmed.

For next-generation sequencing, MiSeq reagent kit v3 manufactured by Illumina, Inc. was used, and MiSeq next-generation sequencer manufactured by Illumina, Inc. was used. The obtained sequence data was read as an FASTQ format text file on the Read1 side and the Read2 side, respectively, and analyzed using Python3 (Python Software Foundation), and DropseqTools and UMITools (open source analysis packages).

For the compartment mixture X, the total number of reads in the second common barcode region (16 bases) and the second unique barcode region (12 bases) was 19,912,682. Furthermore, the number of the first barcode nucleic acid sequences that could correspond to the second common barcode region (16 bases) and the second unique barcode region (12 bases) was 16,354,670. Among them, the number of reads of the first barcode nucleic acid A corresponding to the condition of containing the drug LPS was 85.1% of the total number of the first barcode nucleic acid reads, and the number of reads of the first barcode nucleic acid B corresponding to the condition of containing no drug was 0.8%. A list of the first barcode nucleic acid sequences corresponding to top 100 cells when, after the read error was further corrected, the cells (second common barcode region sequences) were arranged in the descending order of the number of unique reads (second unique barcodes) is indicated in Table 1.

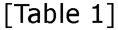

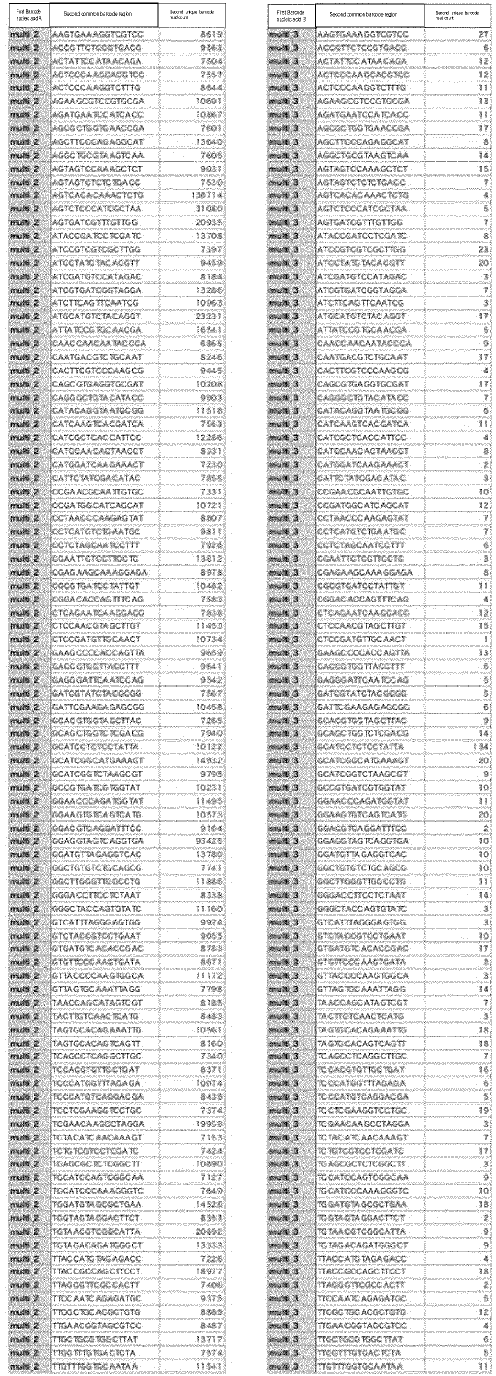

For the compartment mixture Y, the total number of reads in the second common barcode region (16 bases) and the second unique barcode region (12 bases) was 10,795,154, and the number of the first barcode nucleic acid sequences that could correspond to the second common barcode region (16 bases) and the second unique barcode (12 bases) was 7,587,061. Among them, the number of reads of the first barcode nucleic acid A corresponding to the condition of containing the drug LPS was 51.3% of the total number of the first barcode nucleic acid reads, and the number of reads of the first barcode nucleic acid B corresponding to the condition of containing no drug was 35.6% of the total number of the first barcode nucleic acid reads.

Through the series of experiments, it was confirmed that the given conditions to each cell of containing the drug LPS or no drug, which are made to correspond to the DNA barcode type, can be read out for each cell by the one cell gene expression analysis technology using the next-generation genome sequencer.

### REFERENCE SIGNS LIST

101 First barcode nucleic acid
102 First common barcode region
103 First hybridize region
201 Second bead
202 Second barcode nucleic acid
203 Second common barcode region
204 Second unique barcode region
205 Second hybridize region
206 PCR primer region
207 Second barcode nucleic acid linked-bead
301 Test substance 1
302 First barcode nucleic acid corresponding to test substance 1
303 Test substance n
304 First barcode nucleic acid corresponding to test substance n
305 First compartment
306 Cell group
307 Second barcode nucleic acid linked-bead
308 Second compartment
309 Third compartment
310 Cell
312 Cell-derived mRNA
313 Second barcode nucleic acid
314 Hybridized complex
315 Fifth compartment
316 Complementary strand DNA for first barcode nucleic acid
317 cDNA for cell-derived mRNA
318 Amplified product
319 First amplified product
320 Second amplified product
321 Test substance coexisting with cell
322 Transcriptome information of cell
323 Aqueous solvent containing cell lysis buffer
324 Fourth compartment
401 Third bead
402 Third barcode nucleic acid
403 Third common barcode region
404 Third hybridize region
405 Cleavable linker
406 Third barcode nucleic acid linked-bead
501 Test substance 1
502 First barcode nucleic acid corresponding to test substance 1
503 Test substance n
504 First barcode nucleic acid corresponding to test substance n
505 First compartment
506 Cell group
507 Second barcode nucleic acid linked-bead
508 Bead having third barcode nucleic acid
509 Second compartment
510 Third compartment
511 Cell
512 Cell-derived mRNA
513 Second barcode nucleic acid
514 Hybridized complex
515 cDNA for cell-derived mRNA
516 Third barcode nucleic acid
517 Complementary strand DNA for first barcode nucleic acid
518 Amplified product
519 First amplified product
520 Second amplified product
521 Third amplified product
522 Aqueous solvent containing cell lysis buffer
523 Fourth compartment
524 Fifth compartment
525 Test substance coexisting with cell
526 Nondestructive measurement information of cell
527 Transcriptome information of cell
528 cDNA for third barcode nucleic acid
600 Bead having third barcode nucleic acid
601 Third bead
602 Third barcode nucleic acid
603 Third common barcode region
604 Third hybridize region
605 Cleavable linker
610 Second barcode nucleic acid linked-bead
611 Second bead
612 Second barcode nucleic acid
613 Second common barcode region
614 Second unique barcode region
615 Second hybridize region
616 PCR primer region
620 Cell
621 Particular sequence of genome-related nucleic acid
622 Genome-related nucleic acid
623 Test substance 1
624 First barcode nucleic acid corresponding to test substance 1
625 First compartment
626 First hybridize region
627 First common barcode region
630 Third compartment
700 Bead having third barcode nucleic acid
701 Third bead
702 Third barcode nucleic acid
703 Third common barcode region
704 Third hybridize region
705 Cleavable linker
710 Second barcode nucleic acid linked-bead
711 Second bead
712 Second barcode nucleic acid
713 Second common barcode region
714 Second unique barcode region
715 Second hybridize region
716 PCR primer region
720 Nucleic acid probe
721 Molecule binding specifically to molecule such as target protein expressed in cell
722 Fourth barcode nucleic acid
723 Fourth common barcode region
724 Fourth hybridize region
725 Cleavable linker
730 Cell
740 Third compartment
750 First compartment
751 Test substance 1
752 First barcode nucleic acid corresponding to test substance 1
753 First hybridize region
754 First common barcode region
800 Bead having third barcode nucleic acid
801 Third bead
802 Third barcode nucleic acid
803 Third common barcode region
804 Third hybridize region
805 Cleavable linker
806 Particular sequence
807 Primer having sequence complementary to particular sequence
810 Second barcode nucleic acid linked-bead
812 Second barcode nucleic acid
815 Second hybridize region
900 Bead having third barcode nucleic acid
901 Third bead
902 Third barcode nucleic acid
903 Third common barcode region
904 Third unique barcode region
905 Third hybridize region
906 Cleavable linker
907 Acrylamide moiety
908 PCR primer region
910 Second barcode nucleic acid linked-bead
911 Second bead
912 Second barcode nucleic acid
913 Second common barcode region
914 Second unique barcode region
915 Second hybridize region
916 PCR primer region
920 Cell
921 Cell-derived mRNA
922 DNA polymerase
923 Complementary strand DNA
924 cDNA for cell-derived mRNA
925 DNA tag
926 Test substance 1
928 First barcode nucleic acid
929 First common barcode region
930 First hybridize region
931 DNA polymerase
932 Complementary strand DNA
1001 First compartment
1002 Cell
1003 Second barcode nucleic acid linked-bead
1004 Oil
1005 Second compartment
1006 Third compartment
1007 Reservoir
1008 Aqueous solvent containing cell lysis buffer
1009 Oil
1010 Fourth compartment
1011 Fifth compartment
1012 Outlet
1101 First compartment
1102 Cell
1103 Second barcode nucleic acid linked-bead
1104 Bead having third barcode nucleic acid
1105 Oil
1106 Second compartment
1107 Third compartment
1108 Fluorescence imaging measurement
1109 Reservoir
1110 Bright field imaging
1111 Aqueous solvent containing cell lysis buffer
1112 Oil
1113 Fourth compartment
1114 Fifth compartment
1115 Outlet

## Claims

1. A method for detecting genome-related information of a cell or a derivative thereof coexisting with at least one type of test substance, comprising:
preparing a plurality of target compartments containing a cell or a derivative thereof coexisting with the test substance, a first barcode nucleic acid, and a second barcode nucleic acid-linked bead,
wherein the first barcode nucleic acid is preliminarily associated with the test substance, and
the second barcode nucleic acid-linked bead contains a plurality of second barcode nucleic acids hybridizable with a genome-related nucleic acid corresponding to a cell genome or a derivative thereof or the first barcode nucleic acid;
hybridizing each of the genome-related nucleic acid and the first barcode nucleic acid with the second barcode nucleic acid to obtain a hybridized complex;
producing an amplified product derived from the hybridized complex; and
detecting genome-related information in the cell after coexistence with the test substance using an expression pattern of the amplified product as an index.

2. The method according to claim 1, wherein the association of the first barcode nucleic acid with the test substance includes making the test substance coexist with the cell or derivative thereof and the first barcode nucleic acid.

3. The method according to claim 1 or 2, wherein the preparation of the target compartments includes binding a subcompartment that contains the cell or derivative thereof made to coexist with the test substance and the first barcode nucleic acid, to a subcompartment that contains the second barcode nucleic acid-linked bead.

4. A method for detecting genome-related information of a cell or a derivative thereof coexisting with at least one type of test substance, comprising:
preparing a plurality of types of first compartments that contain at least one type of test substance and a first barcode nucleic acid corresponding to the test substance, and a plurality of types of second compartments that contain a cell or a derivative thereof and a second barcode nucleic acid-linked bead,
wherein the second barcode nucleic acid-linked bead is linked to a plurality of second barcode nucleic acids hybridizable with a genome-related nucleic acid corresponding to a cell genome or a derivative thereof or the first barcode nucleic acid;
forming a plurality of types of third compartments in which one first compartment and one second compartment are fused,
wherein the cell or derivative thereof coexists with the test substance in the third compartments;
hybridizing each of the genome-related nucleic acid and the first barcode nucleic acid with the second barcode nucleic acid to obtain a hybridized complex;
producing an amplified product derived from the hybridized complex; and
detecting genome-related information in the cell after coexistence with the test substance using an expression pattern of the amplified product as an index.

5. The method according to any one of claims 1 to 4, wherein
the test substance is identified using an expression pattern of a first amplified product derived from a hybridized complex of the first barcode nucleic acid with the second barcode nucleic acid as an index, and
the genome-related information of the cell is detected using an expression pattern of a second amplified product derived from a hybridized complex of the genome-related nucleic acid with the second barcode nucleic acid as an index.

6. The method according to any one of claims 1 to 5, wherein the genome-related nucleic acid is the genome DNA of the cell, or an RNA derived from the genome DNA of the cell or a cDNA thereof.

7. The method according to any one of claims 1 to 6, wherein each first barcode nucleic acid includes a first common barcode region which is common in same test substance and a first hybridize region hybridizable with the second barcode nucleic acid.

8. The method according to any one of claims 1 to 7, wherein sequence information of the first common barcode region becomes an index for identifying the test substance.

9. The method according to any one of claims 1 to 8, wherein each of the plurality of second barcode nucleic acids linked to the second bead includes a second common barcode region which is in common with each other, a second unique barcode region which can be clearly distinguished from each other, and a second hybridize region hybridizable with the genome-related nucleic acid or the first barcode nucleic acid.

10. The method according to claim 9, wherein sequence information of the second common barcode region becomes an index for identifying the cell or derivative thereof existing in the compartment.

11. The method according to claim 9 or 10, wherein sequence information of the second unique barcode region serves as an index for identifying the genome-related nucleic acid.

12. The method according to any one of any one of claims 1 to 8, wherein the second barcode nucleic acid further includes a PCR primer region.

13. The method according to any one of claims 9 to 12,
wherein the second hybridize region includes the first hybridize region or a nucleic acid complementary to the genome-related nucleic acid.

14. The method according to any one of claims 1 to 3 for integrally detecting nondestructive measurement information and genome-related information of a cell coexisting with at least one type of test substance,
the target compartment further containing a bead having a third barcode nucleic acid,
wherein the bead having a third barcode nucleic acid is:
a particle cleavably linked to a third barcode nucleic acid corresponding to each imaging information or
an organism containing a third barcode nucleic acid corresponding to each imaging information, and
imaging information of the bead having a third barcode nucleic acid can be clearly distinguished from each other,
the method further comprising:
detecting both nondestructive measurement information of the cell and imaging information of the bead having a third barcode nucleic acid and associating the nondestructive measurement information of the cell with the imaging information of the bead having a third barcode nucleic acid;
cleaving or taking out the third barcode nucleic acid from the associated bead having the third barcode nucleic acid, and hybridizing each of the genome-related nucleic acid and the third barcode nucleic acid with the second barcode nucleic acid to obtain a hybridized complex;
producing an amplified product derived from the hybridized complex; and
integrally detecting nondestructive measurement information and genome-related information of the cell using an expression pattern of the amplified product as an index.

15. The method according to any one of claims 4 to 13 for integrally detecting nondestructive measurement information and genome-related information of a cell coexisting with at least one type of test substance,
the second compartment further containing a bead having a third barcode nucleic acid,
wherein the bead having a third barcode nucleic acid is:
a particle cleavably linked to a third barcode nucleic acid corresponding to each imaging information or
an organism containing a third barcode nucleic acid corresponding to each imaging information, and
imaging information of the bead having a third barcode nucleic acid in the third compartment can be clearly distinguished from each other,
the method further comprising:
detecting both nondestructive measurement information of the cell and imaging information of the bead having a third barcode nucleic acid and associating the nondestructive measurement information of the cell with the imaging information of the bead having a third barcode nucleic acid;
cleaving or taking out the third barcode nucleic acid from the associated bead having the third barcode nucleic acid, and hybridizing each of the genome-related nucleic acid and the third barcode nucleic acid with the second barcode nucleic acid to obtain a hybridized complex;
producing an amplified product derived from the hybridized complex; and
integrally detecting nondestructive measurement information and genome-related information of the cell using an expression pattern of the amplified product as an index.

16. The method according to claim 14 or 15, wherein both nondestructive measurement information of the cell and imaging information of the bead having a third barcode nucleic acid are detected, and the nondestructive measurement information of the cell is associated with the imaging information of the bead having a third barcode nucleic acid, before the preparation of the third compartment, in the second compartment and/or the third compartment.

17. The method according to any one of claims 14 to 16, wherein the nondestructive measurement information is based on at least one piece of measurement information selected from color, fluorescence, size, shape, electromagnetic wave, transmission, phase, scattering, reflection, coherent Raman, Raman, and absorption spectrum.

18. A combination of a first barcode nucleic acid and a second barcode nucleic acid-linked bead for detecting genome-related information of a cell coexisting with at least one type of test substance, wherein
each first barcode nucleic acid corresponds to the at least one type of test substance coexisting with the cell;
the second barcode nucleic acid-linked bead is linked to a plurality of second barcode nucleic acids hybridizable with a genome-related nucleic acid corresponding to a cell genome or a derivative thereof or the first barcode nucleic acid; and
the test substance can be identified using an expression pattern of a first amplified product derived from a hybridized complex of the first barcode nucleic acid with the second barcode nucleic acid as an index, and the genome-related information of the cell can be detected using an expression pattern of a second amplified product derived from a hybridized complex of the genome-related nucleic acid with the second barcode nucleic acid as an index.

19. A detecting agent comprising a first barcode nucleic acid, which is used together with a second barcode nucleic acid-linked bead, for detecting genome-related information of a cell coexisting with at least one type of test substance, wherein
each first barcode nucleic acid corresponds to the at least one type of test substance coexisting with the cell;
the second barcode nucleic acid-linked bead is linked to a plurality of second barcode nucleic acids hybridizable with a genome-related nucleic acid corresponding to a cell genome or a derivative thereof or the first barcode nucleic acid; and
the test substance can be identified using an expression pattern of a first amplified product derived from a hybridized complex of the first barcode nucleic acid with the second barcode nucleic acid as an index, and the genome-related information of the cell can be detected using an expression pattern of a second amplified product derived from a hybridized complex of the genome-related nucleic acid with the second barcode nucleic acid as an index.

20. A detecting agent comprising a second barcode nucleic acid-linked bead, which is used together with a first barcode nucleic acid, for detecting genome-related information of a cell coexisting with at least one type of test substance, wherein
each first barcode nucleic acid corresponds to the at least one type of test substance coexisting with the cell;
the second barcode nucleic acid-linked bead is linked to a plurality of second barcode nucleic acids hybridizable with a genome-related nucleic acid corresponding to a cell genome or a derivative thereof or the first barcode nucleic acid; and
the test substance can be identified using an expression pattern of a first amplified product derived from a hybridized complex of the first barcode nucleic acid with the second barcode nucleic acid as an index, and the genome-related information of the cell can be detected using an expression pattern of a second amplified product derived from a hybridized complex of the genome-related nucleic acid with the second barcode nucleic acid as an index.

21. A composition comprising a plurality of types of first compartments, wherein each first compartment contains one type of test substance and a first barcode nucleic acid corresponding to the one type of test substance.

22. A composition comprising a plurality of types of second compartments, which are used together with a first barcode nucleic acid, for detecting genome-related information of a cell coexisting with at least one type of test substance, wherein
each first barcode nucleic acid corresponds to the at least one type of test substance coexisting with the cell;
each second compartment contains a cell or a derivative thereof and a second barcode nucleic acid-linked bead;
the second barcode nucleic acid-linked bead is linked to a plurality of second barcode nucleic acids hybridizable with a genome-related nucleic acid corresponding to a cell genome or a derivative thereof or the first barcode nucleic acid; and
the test substance can be identified using an expression pattern of a first amplified product derived from a hybridized complex of the first barcode nucleic acid with the second barcode nucleic acid as an index, and the genome-related information of the cell can be detected using an expression pattern of a second amplified product derived from a hybridized complex of the genome-related nucleic acid with the second barcode nucleic acid as an index.

23. A method for screening for a test substance, based on the genome-related information of the cell or derivative thereof detected by the method according to any one of claims 1 to 16.

24. A method for screening for a test substance, based on the nondestructive measurement information and the genome-related information of the cell detected by the method according to any one of claims 14 to 16.
